# EUROPEAN PATENT APPLICATION

(11) **EP 1 174 514 A1**
(43) Date of publication of application: **23.01.2002**
(21) Application number: 00115626.4
(22) Date of filing: 20.07.2000
(51) Int. Cl.: C12N 15/86, A61K 48/00, C12N 7/04, A61K 39/145

(54) **Recombinant influenza viruses with bicistronic vRNAs coding for two genes in tandem arrangement**

(71) Applicant: ARTEMIS Pharmaceuticals GmbH, 51063 Köln (DE)
(72) Inventor: Hobom, Gerd, 35392 Giessen (DE); Menke, Anette, 35037 Marburg (DE); Meyer-Rogge, Sabine, 35321 Laubach-Münster (DE)
(74) Representative: Helbing, Jörg, Dr.Dipl.-Chem.

(57) **Abstract**

The invention relates to recombinant influenza viruses for high-yield expression of incorporated foreign gene(s), which are genetically stable in the absence of any helper virus and which comprise at least one viral RNA segment being a tandem bicistronic RNA molecule coding for two genes in tandem, in said tandem bicistronic RNA molecule one of the standard viral genes being in covalent junction with a foreign, recombinant gene and having an upstream splice donor and a downstream splice acceptor signal surrounding the proximal coding region.

The invention further provides a method for obtaining attenuated viruses which resist reassortment dependent progeny production in case of chance superinfections by wild-type influenza viruses; a method for the production of said recombinant influenza viruses; pharmaceutical compositions comprising said recombinant influenza viruses; and the use of said recombinant influenza viruses for preparing medicaments for vaccination purposes.

## Description

### Field of the Invention

The invention relates to recombinant influenza viruses for high-yield expression of incorporated foreign gene(s), which are genetically stable in the absence of any helper virus and which comprise at least one viral RNA segment being a tandem bicistronic RNA molecule coding for two genes in tandem, in said tandem bicistronic RNA molecule one of the standard viral genes being in covalent junction with a foreign, recombinant gene and having an upstream splice donor and a downstream splice acceptor signal surrounding the proximal coding region. In particular the above tandem bicistronic RNA molecule contains one of the standard viral genes in distal mRNA position behind a foreign, recombinant gene in proximal position, or vice versa, both in antisense orientation with regard to the viral RNA within the virus. For simultaneous expression of both genes the proximal reading frame is flanked by splice donor and acceptor signals which have the quality to allow a partial yield of spliced mRNA only, i.e., resulting in the presence of both, spliced and unspliced mRNA simultaneously.

The invention further provides a method for obtaining attenuated viruses which resist reassortment dependent progeny production in case of chance superinfections by wild-type influenza viruses; a method for the production of said recombinant influenza viruses; pharmaceutical compositions comprising said recombinant influenza viruses; and the use of said recombinant influenza viruses for preparing medicaments for vaccination purposes.

### Technical Background

Redesigning influenza virus into a vector system for expression of foreign genes similar to what has been achieved in several other thoroughly studied viruses such as adenovirus, retrovirus, Semliki Forest virus or Rabies virus has the advantage of an industrially well established mode of cheap propagation for influenza in fertilized chicken eggs leading to rather high titers (above 10¹⁰/ml). On the other hand none of the constituent vRNA segments may be deleted from the influenza genome according to our present knowledge, and give room for large-size foreign insertions. Only small fragments of foreign polypeptide chains such as B cell epitopes (10 to 15 amino acids) may be inserted into selected positions within two of the viral proteins, i.e. in exchange for one of the variable antigenic regions located at the surface of hemagglutinin (Muster et al., Mucosal model of immunization against human immunodeficiency virus type 1 with a chimeric influenza virus, J. Virol. 69 (11), 6678-6686 (1995)), or into the stalk sequence of viral neuraminidase (Garcia-Sastre and Palese, The cytoplasmic tail of the neuraminidase protein of influenza A virus does not play an important role in the packaging of this protein into viral envelopes, Virus Res. 37, 37-47 (1995)), and be stably maintained as functional fusion proteins. Constructs of this kind turned out to be useful for experimental vaccination in a few cases studied, but only rather few clearly defined epitope sequences (of ten to twelve amino acids each) are known today, and some of them might also be misfolded within such restricted fusion protein positions, or in other cases interfere with formation of the correct tertiary structure and function of their host polypeptide chains.
Incorporation of a full-size foreign protein into influenza virus via reverse genetics, encoded by an independent ninth vRNA molecule in addition to its regular set of eight standard vRNA segments is without special provisions only transiently possible (Luytjes et al., Amplification, expression, and packaging of a foreign gene by influenza virus. Cell 59, 1107-1113 (1989); Enami et al., An influenza virus containing nine different RNA segments, Virology 185, 291-298 (1991)). In the absence of a continuous selective pressure any additional recombinant vRNA segment cannot be stably maintained as long as the wildtype promoter sequence is used on that ninth vRNA segment, and it will inadvertently be lost after few steps of viral propagation.
Using a different system of influenza reverse genetics developed in our laboratory (Zobel et al., RNA polymerase I catalysed transcription of insert viral cDNA, Nucleic Acids Res. 21, 3607-3614 (1993); Neumann et al., RNA polymerase I-mediated expression of influenza viral RNA molecules, Virology 202, 477-479 (1994)), which was built around *in vivo* synthesis of recombinant vRNA molecules by cellular RNA polymerase I transcription of the respective template cDNA constructs, modified terminal viral RNA sequences (hereinafter "promoter-up mutations" or promoter-up variants") have been designed by nucleotide substitutions (Neumann and Hobom, Mutational analysis of influenza virus promoter elements *in vivo,* J. Gen. Virol. 76, 1709-1717 (1995); WO 96/10641). The above promoter-up variants carry up to five nucleotide substitutions (in promoter-up variant 1920; see Flick and Hobom, J. Gen. Virol. 80, 2565-2572 (1999)). When these promoter-up variants are attached to a recombinant ninth vRNA segment its increased transcription and amplification rate will not only compensate for the losses suffered spontaneously, but even cause accumulation of the foreign vRNA segment during simple viral passaging, in the absence of any selection.

However, due to its over-replication relative to all of the regular influenza vRNA segments (which of course are connected to wild-type promoter sequences) after catching up with the others the foreign segment will become over-abundant. This increasingly will result in viral particles that have incorporated several copies of recombinant vRNA, but no longer have a full set of all eight standard segments incorporated among an average of about 12-15 vRNA molecules present within a virion. Such particles are defective and will not result in plaque formation, hence after an initial increase of recombinant viral particles during the first steps of propagation a dramatic decrease is observed, usually at the third or fourth step of viral passaging, depending on the size of the recombinant vRNA and the level of the promoter-up mutation attached.

A balanced situation with regard to the insert length and the level of promoter activity can be achieved, and has been propagated in a particular case over 11 passages, with essentially stable levels of recombinant viruses among a majority of helper viruses (around 80%) during these steps. If a full-level promoter-up mutation is used (1104 or the variant 1920, see below) a balanced-level propagation is reached in conjunction with a recombinant vRNA size of 4000 nucleotides (Maysa Azzeh, Ph.D. Thesis, Univ. Giessen (2000)).
In all of these preparations, both in transiently achieved increased yields (up to 40% of recombinants after three or four steps of viral passage), and in a balanced propagation of recombinant influenza viruses (10 - 20%) the respective viral progeny inadvertantly constitute mixtures with a majority of non-recombinant helper viruses. These result both from a statistical mode of packaging vRNA molecules into a virion (the ninth segment may not be co-packaged), and from the fraction of cells solely infected by helper virus.
To solve the problems of fractional yields and of instability during viral propagation of recombinant influenza, it was suggested to use a recombinant influenza virus for high-yield expression of incorporated foreign gene(s), which is genetically stable in the absence of any helper virus and which comprises at least one viral RNA segment being an ambisense RNA molecule (designated "ambisense RNA segment") and containing one of the standard viral genes in sense orientation and a foreign, recombinant gene in anti-sense orientation, or *vice versa,* in overall convergent arrangement (PCT/EP00/01903). The ambisense RNA segment preferably should contain the promoter-up mutations. The PCT/EP00/01903 moreover discloses a method of constructing specific influenza carrier (helper) strains carrying one or more ribozyme target sites (of type one) in vRNA flanking positions comprising
(a) RNA polymerase I synthesis of recombinant vRNAs *in vivo,* carrying two different 3' promoter sequences in tandem (an external promoter-up variant and an internal wild-type promoter), which are separated by a second type of ribozyme target sequence, and which carry the said internal ribozyme target sites of type one;
(b) followed by infection of an influenza wildtype strain;
(c) thereafter amplification through simple steps of viral propagation; and
(d) finally isolation through removal of their external 3' promoter sequence by ribozyme cleavage through infection of cells expressing ribozyme type 2, followed by plaque purification.

The resulting special helper virus strains carrying a vRNA segment with external ribozyme target sites of type 1 in exchange for the equivalent regular vRNA molecule are then used for the rescue of ambisense RNA molecules. These are exclusively maintained in the recombinant viruses after passage of viral propagation through ribozyme (type 1) containing host cells, which will destroy the sensitive vRNA molecules of the specially prepared helper viruses.

However, the above ambisense constructs are susceptible to (intra-nuclear) mRNA double-strand formation, which will partially reduce the expression rates of both the ambisense genes, in particular the gene driven by the (weaker) cRNA promoter. The fluctuating extent of this effect made it difficult to bring the expression rate of the influenza gene within the ambisense segment into balance with other influenza genes.
This was the problem to be solved with the present invention.

### Summary of the Invention

Starting out from two observations in this laboratory which are discussed above and which concern two hitherto unsuspected properties of influenza viral RNA polymerase in its interaction with terminally adapted influenza-specific RNA molecules, stable recombinant influenza viruses were found, which solve the above problems.

The recombinant viruses of the present invention can be used for cheap propagation in fertilized eggs, either for production of those recombinant viruses themselves or for production of foreign proteins or glycoproteins encoded by them, and hence find application in (glyco)protein production or in providing vector systems for somatic gene therapy or in being used as vaccination agents.

Thus, the present invention provides
(1) a recombinant influenza virus for high-yield expression of incorporated foreign gene(s), which is genetically stable in the absence of any helper virus and which comprises at least one viral RNA segment being a bicistronic RNA molecule coding for two genes in tandem arrangement (hereinafter "tandem bicistronic RNA segment" or "tandem RNA segment"), in said tandem RNA segment one of the standard viral genes being in covalent junction with a foreign, recombinant gene and said tandem RNA segment having an upstream splice donor and a downstream splice acceptor signal surrounding the proximal coding region;
(2) a preferred embodiment of the recombinant influenza virus defined in (1) above, in which the terminal viral RNA sequences of said at least one tandem RNA segment, which are active as the promoter signal, have been modified by nucleotide substitutions in up to five positions, resulting in improved transcription rates of both the vRNA promoter as well as the cRNA promoter as present in the complementary sequence;
(3) a method for the production of recombinant influenza viruses as defined in (1) and (2) above comprising
   (a) RNA polymerase I synthesis of recombinant vRNAs *in vivo,* in antisense, or in sense tandem design,
   (b) followed by infection with an influenza carrier strain constructed to include flanking ribozyme target sequences in the corresponding viral RNA segment, i.e., coding for the same viral gene as present in the tandem segment distal position, and
   (c) thereafter selective vRNA inactivation through ribozyme cleavage;
(4) a pharmaceutical composition comprising a recombinant influenza virus as defined in (1) and (2) above;
(5) the use of a recombinant influenza virus as defined in (1) and (2) above for preparing a medicament for vaccination purposes;
(6) the use of a recombinant influenza virus as defined in (1) and (2) above for preparing agents for somatic gene therapy;
(7) the use of a recombinant influenza virus as defined in (1) and (2) above for preparing agents for transfer and expression of foreign genes into cells (abortively) infected by such viruses;
(8) the use of a recombinant influenza virus as defined in (1) and (2) above for preparing agents for transfer and expression of RNA molecules into cells infected by such viruses;
(9) a method for the production of proteins or glycoproteins which comprises utilizing a recombinant influenza virus as defined in (1) and (2) above as expression vector;
(10) a method for preventing and/or treating influenza which comprises administering a recombinant influenza virus as defined in (1) and (2) above to the mammal to be treated, i.e., a vaccination method utilizing said recombinant virus;
(11) a method for somatic gene therapy, which method comprises subjecting the organism to be treated with a recombinant influenza virus as defined in (1) and (2) above;
(12) a method for transfer and expression of foreign genes into cells, and for transfer and expression of RNA molecules into cells, which method comprises infecting the cells with a recombinant influenza virus as defined in (1) and (2) above;
(13) use of a recombinant influenza virus as defined in (1) and (2) above for preparing agents for autologous immunotherapy;
(14) a method for an immunotherapy which comprises *ex vivo* infection of immune cells with a recombinant influenza virus as defined in (1) and (2) above, and introduction of the transduced cells into the patient; and
(15) a method for the induction of antibodies which comprises utilizing a recombinant influenza virus as defined in (1) and (2) above as an immunogen.

The invention is described in more detail below.

### Brief Description of the Figures

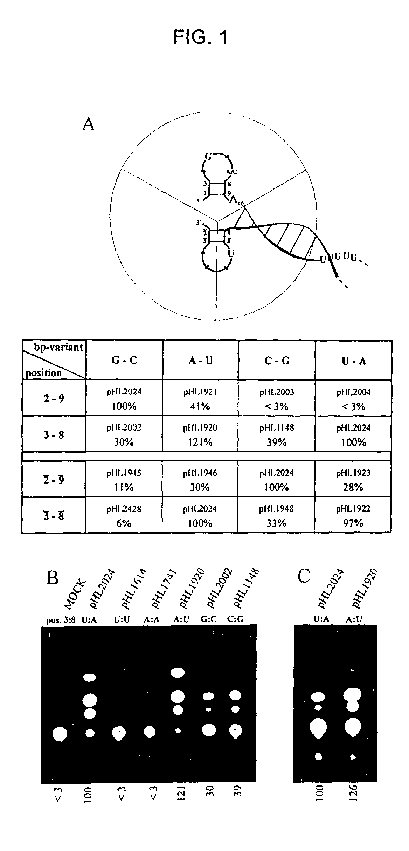

Fig. 1 shows the basepair substitution analysis according to the vRNA 'corkscrew' structure:
(A) 'Corkscrew' conformation of the vRNA promoter drawn against a schematic indication of interacting tripartite viral polymerase. Paired positions exchanged in individual experiments are indicated by numbers, nucleotides 3 or 8 are counted from the 3' end. pHL2024 containing promoter-up mutation '1104' is used as the reference construct (=100%) in all of the CAT assays, while pHL2428 represents the wild-type promoter structure.
(B) CAT analysis of a series of substitution variants in positions 3 and 8 from the 5' end as indicated above the lanes; 50 µl of cell lysate obtained from 10⁶ MDCK cells infected in the first viral passage with recombinant viral progeny.
(C) pHL2024 and pHL1920 comparative CAT analysis, in 100 fold dilution relative to (B), i.e., obtained from 0.5 µl of cell lysate in 3 h reaction time.

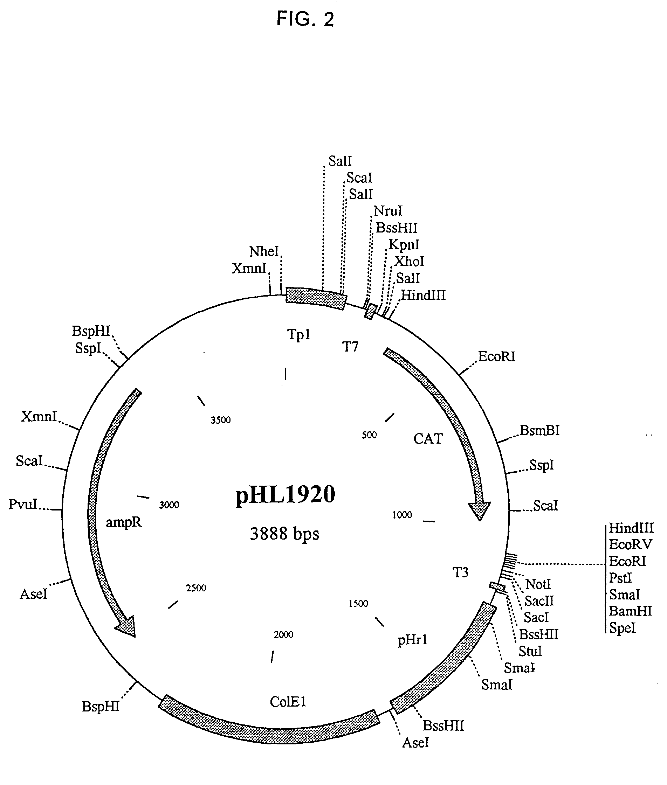

Fig. 2: Vector plasmid pHL1920, the excact sequence of the 3888 bps circular DNA is shown in SEQ ID NO: 20

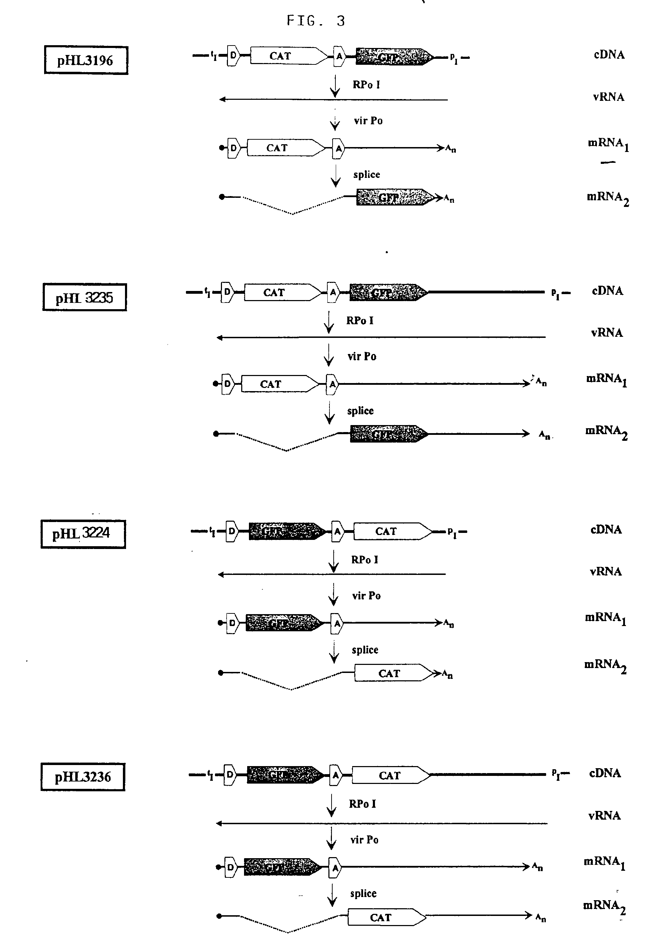

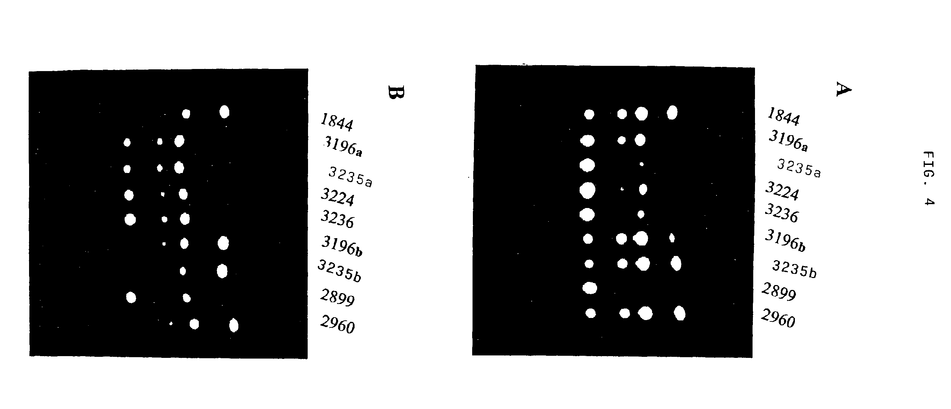

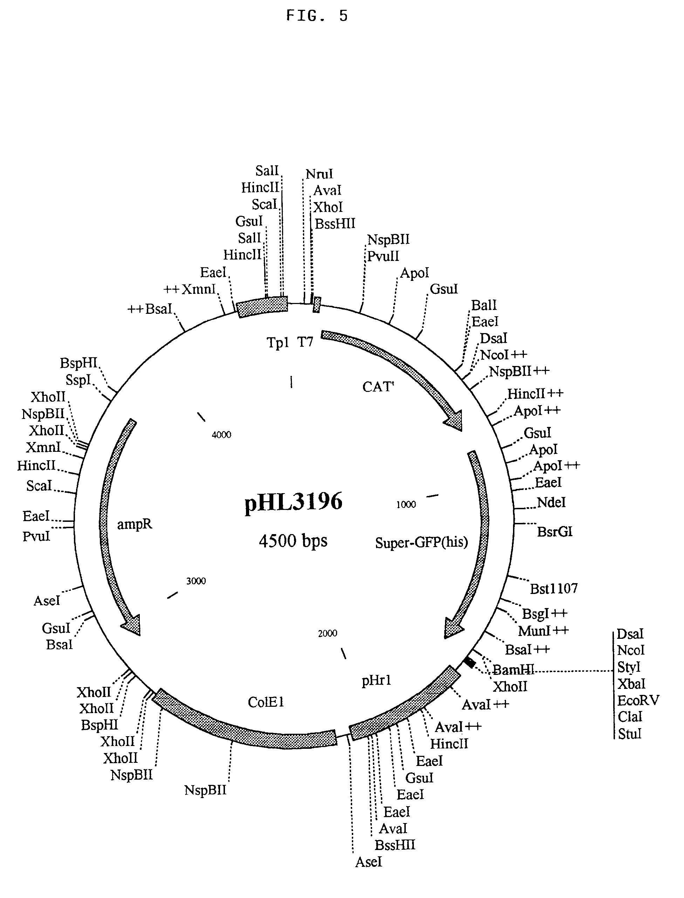

Fig. 3 shows the genetic structure and the RNA transcription products of influenza model tandem expression constructs. Heavy lines for the plasmid cDNA constructs refer to double-stranded DNA, while single-stranded RNA molecules are represented by thin lines, and their 5' to 3' directionalities are marked by arrows. Standard modifications at their 5' and 3' ends are indicated by a dot (5' cap structure) and Aₙ (3' polyadenylation), both are absent in the primary anti-sense transcription product, the viral RNA (vRNA), which is transcribed by cellular RNA polymerase I (RPoI). Full-length mRNA, is synthesized by influenza viral polymerase (virPo), and a partial splice reation results in a functional yield of shorter mRNA₂ molecules. While both of the reporter genes are indicated on the DNA level, together with the positions of splice donor (D) and acceptor (A) signal sequences as well as the promotor (p_{I}) and terminator (t_{I}) elements for RNA polymerase I start and stop, on the RNA level only those genes and splice signals are marked that are actually translated into protein or actively involved in splicing. The chloramphenicol-acetyltransferase gene (CAT) has been inserted in proximal position in pHL3196 and pHL3235, and in distal position in pHL3224 and pHL3236 (see Figs. 5 to 8), while green fluorescent protein (GFP) in each case is located in alternate location. All vRNA molecules - and hence, also the cDNA constructs - carry sequence variations at their 3' ends, which together constitute the 1104 promoter-up mutations: G3A, U5C, C8U (nucleotide positions counted from the 3' vRNA end). pHL3235 and pHL3236 vRNAs are extended in size by about 1000 nucleotides of untranslated sequence relative to pHL3196 and pHL3226: 2600 instead of 1600 nucleotides in lengths. For full-size representation of circular plasmid DNAs see Figs. 5-8, for CAT expression data of all infected by recombinant influenza viruses carrying the respective viral RNAs see Fig 4.

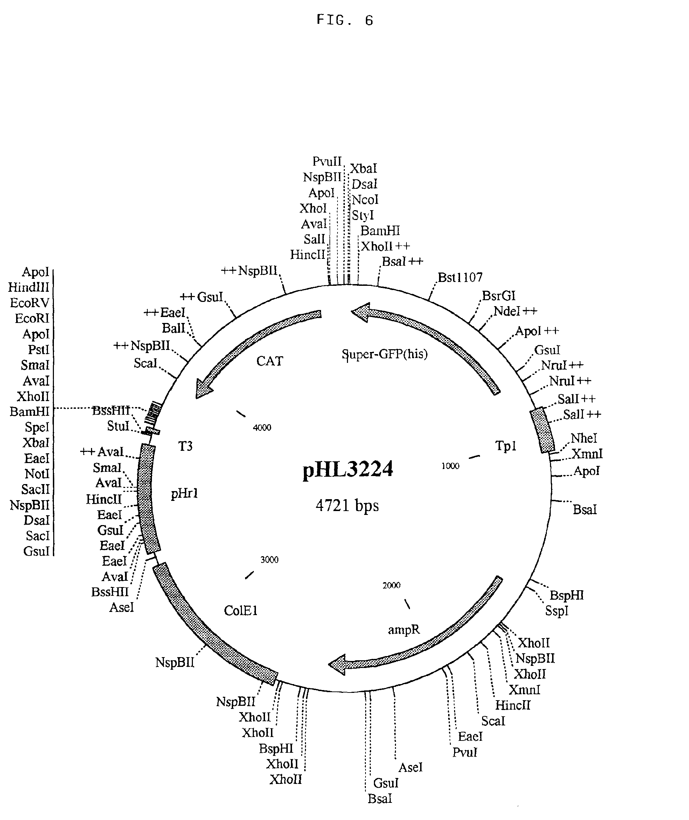

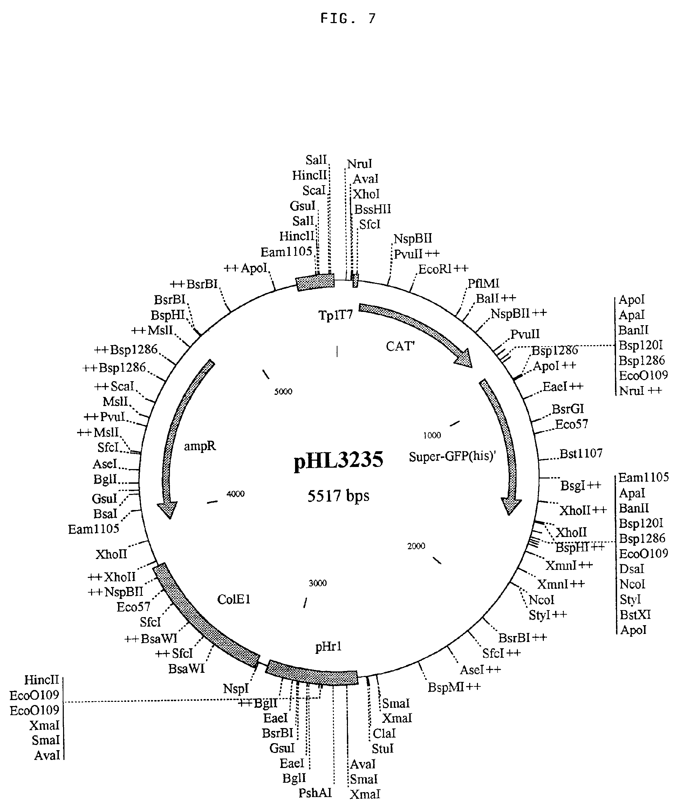

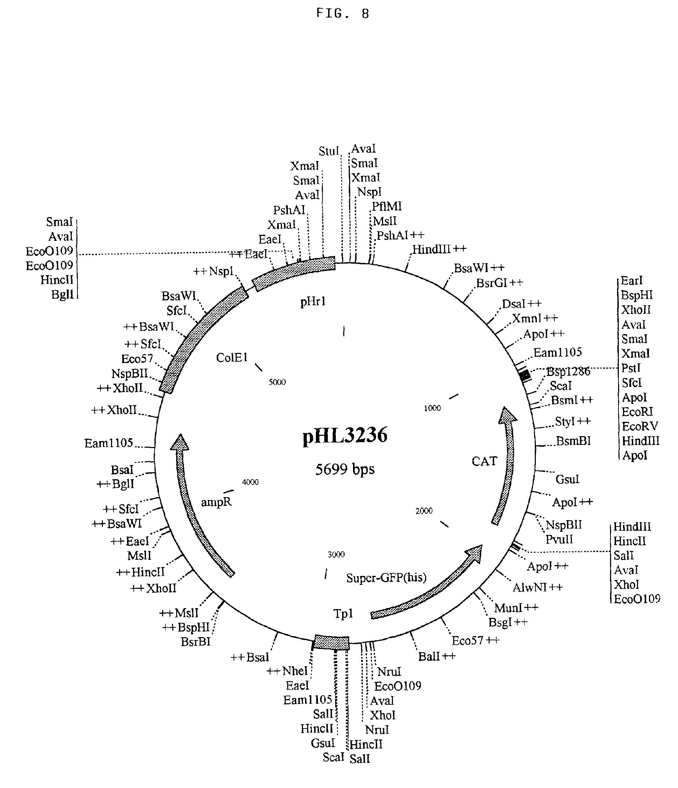

Fig. 4 shows the CAT assay results for the group of tandem vRNA plasmid constructs as described in the Example. In particular, the ratio between chloramphenicol (bottom line) and acetylchloramphenicol (upper three lines) in a flash-CAT assay, after the 2^{nd} (A) and 4^{th} (B) passage of recombinant viruses carrying the reportergene CAT, can be determined from said figure. The following constructs were utilized:
pHL1844 (control): monocistronic CAT-construct downstream of promoter variant 1104.
pHL3196: tandem construct, p-CAT-GFP resulting in a vRNA having a total length of 1530 nucleotides (not "extended"), see also Fig. 5.
pHL3235: tandem construct, p-CAT-GFP resulting in a vRNA having a total length of 2550 nucleotides ("extended"), see also Fig. 7.
pHL3224: tandem construct, p-CAT-GFP resulting in a vRNA having a total length of 1700 nucleotides (not "extended"), see also Fig. 6.
pHL3236: tandem construct, p-CAT-GFP resulting in a vRNA having a total length of 2720 nucleotides ("extended"), see also Fig. 8.
pHL2899: ambisense construct, pᵥ-CAT→ ←GFP-p_{c} resulting in an RNA having a total length of 1500 nucleotides.
pHL2960: ambisense construct, pᵥ-CAT→ ←GFP-p_{c} resulting in an RNA having a total length of 1500 nucleotides.

The five constructs on the left side were transfected into the cell DNA without the use of "booster" plasmides, the four constructs on the right side were, however, transfected with the "booster" plasmides, which gives a jump-start of the constructs due to recombinant vRNA amplification prior to helper virus injection, equivalent to an advantage of about two passages. The "booster" plasmides comprise expression constructs for the nucleoprotein as well as the three subunits of influenza viral polymerase, each downstream of an RNA polymerase II promoter and in an mRNA forming cassette.
While the ambisense construct having the CAT-reporter gene in the weaker position, i.e. behind the cRNA promoter (pHL2899), is only expressed moderately, this is not the case in the respective tandem construct having the CAT-reporter gene in the weaker position, viz. pHL3224 or pHL3236. Further, the "extension" of the vRNA by 1020 non-translated nucleotides (at the 3' end) is tolerated without significant decrease of expression (see pHL3235 versus pHL3196).

Fig. 5: Vector plasmid pHL3196, the exact sequence of the 4500 bps circular DNA is shown in SEQ ID NO:21.

Fig. 6: Vector plasmid pHL3224, the exact sequence of the 4721 bps circular DNA is shown in SEQ ID NO:22.

Fig. 7: Vector plasmid pHL3235, the exact sequence of the 5517 bps circular DNA is shown in SEQ ID NO:23.

Fig. 8: Vector plasmid pHL3236, the exact sequence of the 5699 bps circular DNA is shown in SEQ ID NO:24.

### Detailed Description of the Invention

According to the present invention "influenza virus" embraces influenza A virus, influenza B virus and influenza C virus, with influenza A virus being preferred.

"Bicistronic" according to the present invention refers to a viral RNA segment, vRNA, cRNA or mRNA that includes two independent genes in covalent junction; in a preferred version one of these genes is of viral origin, while the other one codes for a foreign, recombinant gene product.

"Proximal" and "proximal position" according to the present invention refers to the 5' portion of one of the genes in the bicistronic viral mRNA, i.e., ahead (upstream) of the second gene in "distal position".

A "mammal" according to the present invention includes humans and animals. "Organism" embraces prokaryotic and eukaryotic systems as well as multicellular systems such as vertebrates (including mammals) and invertebrates.

"Infected cells" and "infecting cells" according to the present invention also include "abortively infected cells" and "abortively infecting cells", respectively.

In a preferred influenza virus according to embodiment (1) at least one of the regular viral RNA segments is replaced by a tandem RNA segment which contains one of the standard viral genes in distal position, and a foreign, recombinant gene in proximal position, both in anti-sense orientation, or vice-versa. It is moreover preferred that in the tandem RNA molecule said foreign recombinant gene is covalently bound to one of the viral genes while the original vRNA segment coding for the same gene is deleted from the recombinant virus by specific ribozyme cleavage.

The foreign gene(s) in tandem covalent junction with the viral gene(s) preferably code for proteins and/or glycoproteins which are secreted from cells infected with the recombinant virus, such as lymphokines, or code for glycoproteins that are incorporated into the virion as well as the plasma membrane of the infected cell. In another preferred embodiment the foreign gene(s) in tandem covalent junction with the viral gene(s) code for proteins or artificial polypeptides designed to support an efficient presentation of inherent epitopes at the surface of infected cells, for stimulation of B cell and/or T cell response. Such proteins or artificial polypeptides constitute for instance a tumor antigen or an artificial oligomeric series of T cell epitopes that have been identified within a polypeptide chain. Finally, the foreign gene(s) may be suitable for transfer and expression of RNA molecules, including antisense RNAs and ribozymes, into cells. Such recombinant influenza viruses are suitable for sequence specific gene silencing, for example by antisense or RNA interference mechanisms.

A preferred recombinant virus of the invention is where in the regular viral RNA segments one or both of the standard glycoproteins hemagglutinin and neuraminidase have been exchanged, preferably into fusion glycoproteins consisting of an anchor segment derived from hemagglutinin and an ectodomain obtained from the foreign source, viral or cellular, or in which such recombinant glycoprotein has been inserted as a third molecular species in addition to the remaining standard components.

As set forth in embodiment (2) above, a preferred recombinant virus of the invention is where the terminal viral RNA sequences, which are active as promoter signal, have been modified by nucleotide substitution in up to 5 positions, resulting in improved transcription rates (of both the vRNA promoter and in the cRNA promoter as present in the complentary sequence) as well as enhanced replication and/or expression rates relative to the wild-type sequence. Said modified terminal viral RNA sequences differ from the wild-type sequence in that in said tandem vRNA segment the 12 nucleotide conserved influenza 3' terminal sequence has been modified by replacement of one to three nucleotides occurring in said sequence at positions 3, 5 and 8 relative to the 3' end by other nucleotides provided that the nucleotides introduced in positions 3 and 8 are forming a base pair (i.e., if the nucleotide position 3 is G, than that in position 8 is C; if the nucleotide in position 3 is C, than that in position 8 is G; etc.).

The 3' conserved regions of the wild-type influenza virus have the following sequences:

Influenza A: (5')-CCUGCUUUUGCU-3'
Influenza B: (5')-NN(C/U)GCUUCUGCU-3'
Influenza C: (5')-CCUGCUUCUGCU-3'.

Moreover, the 13 nucleotide conserved influenza 5'-terminal sequence may be modified by replacement of one or two nucleotides occurring in said sequence as positions 3 and 8 by other nucleotides, again provided that the introduced nucleotides are forming a base pair. The 5' conserved regions of the wild-type influenza virus have the following sequences:

Influenza A: 5'-AGUAGAAACAAGG
Influenza B: 5'-AGUAG(A/U)AACA(A/G)NN
Influenza C: 5'-AGCAGUAGCAAG(G/A):

Preferred influenza viruses of the invention are those wherein in the 3' conserved region the replacements G3A and C8U have been performed, more preferred are those where also the replacement U5C has been performed (the above mutations are annotated relative to the 3' end; such counting from the 3' end is also indicated by a line on top of the digit, e.g., G $\overline{\text{3}}$A). Another preferred influenza virus mutant comprises the 3'-terminal nucleotide sequence G3C, U5C and C8G (relative to the 3' end) resulting in the following 3' terminal nucleotide sequence (5')-CCUGGUUCUCCU-3'. Among the influenza viruses defined hereinbefore those having a 3'-terminal nucleotide sequence of (5')-CCUGUUUCUACU-3' are most preferred. In case of an influenza A virus the segment may further have the modifications U3A and A8U in its 5' terminal sequence, in case of influenza C it may have the modifications C3U and G8A in its 5' terminal sequence. The most preferred influenza viruses of the present invention comprise the following general structures:

Influenza A (mutant pHL1104):
5'-AGUAGAAACAAGGNNNU5-6..(880-2300 ntds)..N'N'N'CCUGUUUCUACU-3'
Influenza A (mutant pHL1920):
5'-AGAAGAAUCAAGGNNNU5-6..(880-2300 ntds)..N'N'N'CCUGUUUCUACU-3'
Influenza A (mutant pHL1948):
5'-AGUAGAAACAAGGNNNU5-6..(880-2300 ntds)..N'N'N'CCUGGUUCUCCU-3'

Influenza B:

5'-AGUAG(A/U)AACA(A/G)NNNNNU5-6..(880-2300 ntds)..N'N'N'N'N'(C/U)GUUUCUACU-3'

Influenza C:

5'-AGUAGUAACAAG(G/A)GU5-6..(880-2300 ntds)..CCCCUGUUUCUACU-3'

In the above structures the variables are defined as follows:
(1) Underlined and enlarged letters show the required mutations relative to the wild-type sequence for preparing a promoter mutant with enhanced properties;
(2) enlarged A in position 10 in the 5'-part of the sequence: unpaired A residue, bulge-forming;
(3) (A/G) in one position: different isolates or single segments with variable sequence at the respective position, which are functionally interchangeable;
(4) N and N': positions undefined, but base-paired relative to each other because of complementarity between the 5' and 3' termini, different among the 8 segments, but constant for each segment throughout all viral isolates;
(5) (880-2300 ntds): the lengths of the viral RNA segments, in case of segments with foreign genes increased up to 4,000 nucleotides.

According to embodiments (1) to (3) the invention provides
- a stable recombinant influenza virus containing (up to) seven regular vRNA segments plus one (or more) additional bicistronic segment(s) coding for a foreign gene in covalent conjunction with one of the influenza genes, in tandem arrangement, and
- a method for the construction of stable recombinant influenza viruses through tandem arrangement of bicistronic vRNA segments, which is also applicable as a method for attenuation and for prevention of reassortment between co-infecting influenza viruses.

Expression of both gene products in these constructions is made possible by way of an upstream splice donor and a downstream splice acceptor signal surrounding the proximal coding region of such a quality that splicing does occur in part of the mRNA molecules only, i.e., both mRNAs spliced and unspliced are present in the infected cell. For compensation with regard to the vRNA length the bicistronic segment is connected to a promoter variant of enhanced replication and transcription rates as defined herein before.

The splice donor and the splice acceptor signals are selected from authentic sequences as present in influenza segments 7 and 8 or other partially effective splice reaction substrates, preferably those of influenza virus WSN segment 7, i.e., 5'-AG↓GTACGTTC-3' (donor) and 5'-GCTGAAAAATGATCTTCTTGAAAATTGCAG↓GC-3' (acceptor).

In a particular application of embodiments (1) to (3) the tandem bicistronic mRNA codes for one of the viral genes, such as hemagglutinin, in conjunction with all or part of the viral neuraminidase coding sequence, in antisense orientation, while the authentic neuraminidase vRNA segment is missing in these recombinant viruses. In another variation of these constructs an anti-neuraminidase ribozyme sequence is also provided together with the (partial) neuraminidase antisense sequence, in the proximal position of these bicistronic recombinant segments. Recombinant viruses of this character are propagated in culture media with addition of exogenous neuraminidase.

The absence of a functional neuraminidase gene serves as a strong attenuation mechanism resulting in single-step infections of such recombinant viruses only. While a functional neuraminidase gene could be provided through another (wildtype) influenza virus superinfecting the same cell, expression of that gene is very much reduced through antisense RNA interaction and/or destruction of the corresponding vRNA through ribozyme cleavage, designed to interfere with production of infectious progeny even from co-infected cells; as a barrier against reassortment in double infected cells.

Recombinant viral RNAs coding simultaneously for two genes in tandem in a construct in which one of the viral genes is in covalent junction with a foreign coding sequence, are constructed via E. coli plasmid vector DNAs designed for an *in vivo* transcription of minus-strand vRNAs by cellular RNA polymerase I. In these constructs the gene in plus-strand proximal (upstream) position is surrounded by splice signals of limited activity such that both mRNAs, spliced and unspliced are present in the infected cell. Either the foreign gene or the viral gene may be in that upstream position. In the majority of applications the higher rates of expression will be reserved for the foreign coding sequence, while the lower expression rate of the viral gene is adapted to be approximately in balance with expression of the other viral genes encoded by the regular viral segments.

To achieve such a balanced rate of expression, the splice signals and the promoter have to be chosen properly (Flick and Hobom, Interaction of influenza virus, polymerase with viral RNA in the 'corkscrew' conformation, J. Gen. Virol. 80, 2565-2572 (1999)). At an increased overall transcription rate, the resulting mRNAs shall be spliced inefficiently if the viral gene is in the distal (downstream) position. Vice-versa, if the foreign gene is in the distal position, splicing to obtain the foreign mRNA shall be achieved efficiently. Both designs serve to reach an over-expression of the foreign gene relative to the viral gene, of which the expression shall be in balance with the expression of the other viral genes. Further, the promoter variant attached to the bicistronic segment has the function to compensate for the increased gene length by way of an increased replication rate.

The influenza vRNA segments preferably used for construction of bicistronic segments include the neuraminidase (No. 6), hemagglutinin (No. 4) and NS segment (No. 8). In the NS segment the foreign gene may also substitute for the NS1 gene leaving the viral NS2 gene in its place. These recombinant viruses can, as an example, be made by the following procedure: A recombinant virus population can be selected by repeated ribozyme-mediated cleavage of helper-virus segments carrying ribozyme cleavage sites that flank the same viral gene in the monocistronic segment as is present in the bicistronic construct (PCT/EP00/01903). By serial viral passaging and relying on the ouptut of reporter genes in equivalently constructed bicistronic segments, a balanced mode of expression can be achieved in choosing the right set of elements: promoter, splice signals plus a limited variation in segment length. The construct that gives rise to the balanced, stable expression is then used as a basis for a multiple cDNA transfection procedure in a helper-virus free design according to Neumann et al., Proc. Natl. Acad. Sci. USA, Vol 96, 9345-9350 (August 1999). The resulting recombinant influenza virus, obtained via single plaques in pure helper-free state is subjected to another series of propagation steps to finally evaluate its properties.

In a particular application this design is used for a controlled mode of viral attenuation. Attenuation of influenza viruses so far has been achieved in cold-sensitive mutants (Edwards et al., J. Infect. Dis. 169, 68-76(1994)), by deletion of the NS1 gene (partial attenuation, Egorov et al., J. Virol. 72, 6437-6441 (August 1998) and Palese et al., Proc. Natl. Acad. Sci USA, 4309-4314 (April 2000)), or through deletion of the neuraminidase gene (full attenuation, Kawaoka et al., J. Virol. 74, 5206-5212 (June 2000)). The latter approach is adapted here using a novel technique for the attenuation, which for the first time is also able to interfere with (chance) superinfection by wild-type viruses.

In this embodiment of the invention a bicistronic cDNA construct is achieved, which instead of a foreign gene is coding either for part of or for the entire viral neuraminidase gene in antisense orientation, with or without being surrounded both by splice donor and acceptor elements. In another version of that design a 2 x 50 nucleotide antisense segment complementary to the 5'-terminal neuraminidase sequence has been cloned in flanking positions relative to a ribozyme construct according to the hammerhead design and oriented against a common GUC triplett within the neuraminidase sequence. In a preferred design this antisense expression construct has been attached to the hemagglutinin vRNA segment, while another gene or reporter gene is encoded in a second bicistronic vRNA, in conjunction with NS2.

Propagation of recombinant viruses deleted for the neuraminidase (NA) gene requires an addition of external neuraminidase to the medium. In the absence of neuraminidase, infection by the NA deletion viruses is abortive: no infectious progeny is produced. Upon co-infection (3:3) of recombinant viruses together with wildtype viruses no progeny virus or plaque is observed, which is attributed to antisense-blocked expression or (partial) destruction of the neuraminidase segment originating from the wild-type virus. Therefore, the recombinant viruses described are not only attenuated in single infections, but simultaneously interfere with wildtype virus superinfection, and therefore, no re-assortment between the two viruses will occur.

The pharmaceutical composition according to embodiment (4) above and the medicament of embodiment (5) above contain the recombinant influenza virus in a pharmaceutically effective amount. Besides said recombinant influenza virus, the pharmaceutical composition and the medicament may contain further pharmaceutically acceptable carrier substances well-known to a person skilled in the art, such as binders, desintegrants, diluents, buffers, preservatives, etc. The pharmaceutical composition and medicaments are solid or liquid preparations and are suitable to be administered orally, intravenously or subcutaneously.

The medicament according to embodiment (5) above is preferably suitable as a medicament against influenza and/or against other infections. The recombinant influenza virus may be present in form of inactivated preparations or may be present in form of live recombinant viruses, preferably as attenuated viruses.

Live recombinant viral vaccines, live but attenuated recombinant viral vaccines or inactivated recombinant viral vaccine can be formulated. Inactivated vaccines are "dead" in the sense that their infectivity has been destroyed. Ideally, the infectivity is destroyed without affecting its immunogenicity. To prepare inactivated vaccines, the recombinant virus may be grown in cell cultures or in embryonated chicken eggs, purified, and inactivated by formaldehyde or β-propiolactone. The resulting vaccine is usually administered intramuscularly.

Inactivated viruses may be formulated with suitable adjuvants to enhance the immunological response. Such adjuvants include, but are not limited to, mineral gels, e.g., aluminum hydroxide, surface-active substances such as pluronic polyols, lysolecithin, peptides, oil emulsions, and potentially useful human adjuvants such as BCG.

Many methods may be used to introduce the vaccine formulations above, for example the oral, intradermal, intramuscular, intraperitoneal, subcutaneous, or intranasal routes. Where a live recombinant virus vaccine is used, it is preferred to introduce the formulation via the natural route of infection for influenza virus.

The medicament according to embodiment (5) above is preferably suitable for prophylactic or therapeutic vaccination, or both, against influenza and other infections. For example, recombinant viruses can be made for use in vaccines against HIV, hepatitis B virus, hepatitis C virus, herpes viruses, papilloma viruses, to name but a few. In one embodiment the recombinant virus contains the genes for surface proteins of the viruses, in another the genes for non-structural or regulatory genes. The recombinant viruses may be present in form of inactivated preparations or may be present in form of live recombinant viruses, or as live, but attenuated viruses. In an attenuated virus the recombinant virus would go through a single or at most very few propagation cycle(s) and induce a sufficient level of immune response, but would not cause disease. Such viruses lack one of the essential influenza genes or contain mutations to introduce temperature sensitivity.

The agents of embodiments (6)-(8) of the invention are applicable in *ex vivo* and *in vivo* application schemes. The RNA molecule to be expressed by means of the agent of the embodiment (8) is of an antisense sequence or double strand sequence (in ambisense bidirectional transcription) relative to a target cellular mRNA molecule. In embodiment (8) the agent is preferably suitable for sequence-specific gene silencing, preferably by antisense RNA or RNA interference mechanisms.

The method for the production of proteins or glycoproteins is preferably performed in cell culture cells or in fertilized chicken cells in accordance with standard techniques within the general knowledge of a person skilled in the art. The proteins or glycoproteins to be expressed are those incorporated into the ambisense construct as defined hereinbefore.

The methods according to embodiments (9) to (12), (14) and (15) of the invention include the administration of an effective amount to the mammal or the administration of a sufficient infective dose of the recombinant virus to the cell system that is used for ex vivo therapy or for in vitro investigations, whereby the amount and dose will be determined by a person skilled in the respective arts or knowledgeable of the desired treatments.

The agent of embodiment (14) of the invention is preferably utilized to infect, transfect or transduce patient-derived immune cells. The agent is suitable for treatment of cancer or chronic viral infections. For this purpose, patient derived immune cells, preferably dendritic cells, are *ex vivo* infected with recombinant influenza viruses expressing, e.g., tumor antigens or viral antigens. The transduced cells are then reintroduced into the patient.

The preferred method for immunotherapy of embodiment (14) of the invention is an autologous immunotherapy, wherein the cells which are *ex vivo* infected are patient-derived and the transduced cells are reintroduced into the patient. The diseases to be treated by this method include cancer and chronic viral infections. For details regarding such treatment see discussion of embodiment (13) above.

The method for inducing antibodies according to embodiment (15) of the invention is suitable for inducing antibodies to foreign proteins including glycoproteins, following the administration of protein or glycoprotein antigens as part of a recombinant influenza virus in an authentic conformation, whereby the virus is purified by gentle procedures based on hemagglutination, and the gene is expressed at high rates in the infected cells.

As influenza viruses have a wide host range, recombinant influenza viruses can be used to obtain strong immune responses in, and isolate antibodies from, a wide range of animals, including, but not limited to, fowl, pigs, horses, and mice. Further, influenza viruses adapted to the mouse can be used for the infection of mice by several routes including the intranasal route. This results in infection of the pharyngeal mucosal cells and results in an additional type of B cell response (e.g., as recognized in the ratio of IgG to IgA). Mice are of particular utility in the induction of immune responses in transgenic mice that have been engineered to express human antibodies. As gentle procedures based on hemadsorption are used to purify influenza viruses, antibodies to antigens in native conformation can be isolated from the infected mammals.
The preset invention further illustrated by the following, non-limiting Example:

### Example

Model tandem bicistronic expression constructs using reporter genes CAT and GFP.
Objective: Measurements of relative expression rates for CAT in proximal and distal position, with live observation of GFP fluorescence in alternate position during propagation of recombinant influenza viruses.

### a) Construction of bicistronic expression plasmid DNAs:

Starting out with vector plasmid pHH10 (Hoffmann, Ph.D. Thesis, Univ. Giessen (1997)), i.e. an ampicillin resistant plasmid including in between a human rDNA promoter segment and a murine rDNA terminator segment precisely inserted cDNA sequence elements representing the 5' and 3' vRNA sequence of influenza rRNA segment 5, and finally a central multiple cloning site sequence as obtained from plasmid PBSK, both reporter genes have been inserted in a stepwise manner. After that, to the proximal reading frame, i.e. CAT in pHL3196, and GFP in pHL3224, has been added an upstream splice donor sequence element and a downstream splice acceptor element, both inserted as double-strand oligonucleotides, in between particular restriction cleavage sites available in the respective positions. The signal sequences used in that pair of plasmids indicated above have been derived from influenza vRNA segment 7, which is known for its partial splice reactions yielding both gene products, M1 and M2, simultaneously. By insertion of a non-transcribed DNA fragment (representing an internal segment of the influenza PB1 coding region) in a distal position relative to both reading frames, pHL3196 has been converted into pHL3235, and pHL3224 into pHL3236. For the resulting plasmid constructs see Figs 5-8 and SEQ ID NOs: 21-24.

### b) Transfection of plasmid DNAs and isolation of recombinant influenza viruses:

Semi-confluent 293-T cells, a human renal cacinoma cell line carrying an artificially integrated tumor virus SV40 T-antigen gene, were- DNA-transfected using lipofectamine: 5-10 µg of DNA mixed with 30µl Lipofectamine® (GIBCO/BRL) were added to 370 µl of DMEM medium and were incubated with 5x10⁶ to 10⁷ cells, washed and maintained serum-free for 5 to 8 hours, before serum was added for another 12 to 15 hours. Finally influenza helper virus FPV_{Bratislava} was used for infection of the DNA-transfected cells. The supernatant containing a mixture of helper viruses and recombinant viruses was collected for further propagation after 8 to 12 hours of infection, while the sedimented cells were used for preparation of a cell lysate, fractions of which were inserted in the CAT assay procedure.
Viral propagation was achieved by infection of MDCK cells (Madin-Darby canine kidney cell line) again in semi-confluent state (5x10⁶ to 10⁷ cells per plate), generally using 1ml of the previous supernatant for infection. Serial propagations were done in the same way, with preparation of cell lysates for CAT assays at the end of each step. Infected cells were also used for observation of GFP fluorescence.

### c) CAT assay:

Bacterial chloramphenicol-acetyltransferase (CAT) is accumulated in eukaryotic cells without degradation and can be used for representative gene expression measurements. The substrate used here is fluorescent boron-dipyrromethane-chloramphenicol diflouride (FLASH CAT-KIT® ; Stratagene). 50 µl of cell lysate or reduced/diluted samples thereof were used for incubation with 7.5 µl of fluorescent substrate and 10 µl acetyl-CoA (4mM) co-substrate in 19 mM Tris/HCl, pH: 7.5 at 37°C for 3 hours. For extraction of reaction products 1ml of ethylacetate ins added, the mixture is vortexed, and separated by centrifugation. After solvent evaporation and dissolution again in 20ml ethylacetate, the reaction products are separated on a silica thin-layer chramatography plate using chloroform/methanol 87:13% (vol.) and the results are documented by photography under UV light.

### d) Results

CAT in proximal or in distal position of this pair of recombinant plasmids is expressed about equally (Fig.4), and the same is true for GFP (not shown). The expression rates are increasing during the initial steps of viral propagation and stay about constant afterwards during further steps of recombinant viral passages, different from expression rates in ambisense bicistronic constructs (pHL2899 and pHL2960) (Fig. 4B). Co-transfection of booster plasmids in the initial 293-T cells increase the yields of recombinant viruses within the progeny population, which are maintained during consecutive steps of propagation. Addition of 1000 nucleotides of untranslated vRNA sequence will not reduce the expression rates substantially (pHL3235 versus pHL3196, and pHL3236 versus pHL3224).

## Claims

1. A recombinant influenza virus for high-yield expression of incorporated foreign gene(s), which is genetically stable in the absence of any helper virus and which comprises at least one viral RNA segment being a bicistronic RNA molecule coding for two genes in tandem arrangement (tandem RNA segment), in said tandem RNA segment one of the standard viral genes being in covalent junction with a foreign, recombinant gene and said tandem RNA segment having an upstream splice donor and a downstream splice acceptor signal surrounding the proximal coding region.

2. The recombinant influenza virus of claim 1, wherein the tandem RNA segment contains one of the standard viral genes in distal mRNA position behind a foreign, recombinant gene in proximal position, or vice versa, both in antisense orientation with regard to the viral RNA as present within the virus.

3. The recombinant influenza virus of claim 1 or 2, wherein at least one of the regular viral RNA segments is replaced by a tandem RNA segment, preferably the replaced regular viral RNA segment is selected from the neuraminidase segment, hemaglutinin segment and NS segment.

4. The recombinant influenza virus of claims 1 to 3, wherein the splice donor and splice acceptor signals are selected from sequences as present in influenza WSN segment 7 and 8 or other partially effective splice reactin substrates.

5. The recombinant influenza virus of claim 4, wherein the splice donor and splice acceptor signals are selected from sequences as present in influenza WSN segment 7.

6. The recombinant influenza virus according to claims 1 to 5, wherein one or more of the regular viral RNA segments, differing from said at least one tandem RNA segment, comprises a vRNA encoding a foreign gene which may or may not be in covalent connection to one of the viral genes, and preferably one or more of the regular viral RNA segments has (have) been deleted and replaced by a tandem vRNA encoding in addition a foreign gene.

7. The recombinant influenza virus according to claims 1 to 6, in which the terminal viral RNA sequences of one or more of the regular segments and/or of the at least one tandem RNA segment, which are active as the promoter signal, have been modified by nucleotide substitutions in up to five positions, resulting in improved transcription rates of both the vRNA promoter as well as the cRNA promoter as present in the complementary sequence.

8. The recombinant influenza virus of claim 7, wherein the 12 nucleotide conserved influenza 3' terminal sequence has been modified by replacement of one to three nucleotides occurring in said sequence at positions 3, 5 and 8 relative to the 3' end by other nucleotides, and/or wherein the 13 nucleotide conserved influenza 5' terminal sequence has been modified by replacement of one or two nucleotides occurring in said sequence at positions 3 and 8 by other nucleotides.

9. The recombinant influenza virus of claim 8, wherein the replacements in the 3' terminal nucleotide sequence comprises the modifications G3A and C8U.

10. The recombinant influenza virus of claim 9, wherein the replacements in the 3' terminal nucleotide sequence comprises the modifications G3A, U5C and C8U, or G3C, U5C and C8G.

11. The recombinant influenza virus of claim 10, which comprises a 3' terminal nucleotide sequence of (5')-CCUGUUUCUACU-3'.

12. The rcombinant influenza virus according to claims 7 to 12, wherein the 5' terminal nucleotide sequence comprises the modifications U3A and A8U resulting in a 5'-terminal sequence of 5'-AGAAGAAUCAAGG.

13. The recombinant influenza virus according to claims 1 to 12, which is a recombinant influenza A virus.

14. The recombinant influenza virus according to claims 1 to 13, in which the foreign gene(s) in the tandem RNA segment code for proteins and/or glycoproteins which are secreted from cells infected with the recombinant virus.

15. The recombinant influenza virus according to claims 1 to 13, in which the foreign gene(s) in the tandem RNA segment code for proteins or artificial polypeptides designed to support an efficient presentation of inherent epitopes at the surface of infected cells, for stimulation of a B cell and/or T cell response.

16. The recombinant influenza virus according to claims 1 to 13, in which the foreign gene(s) in the tandem RNA segment is a nucleotide sequence causing viral attenuation.

17. The recombinant influenza virus of claim 16, wherein the foreign gene is coding for part of or for the entire viral neuraminidase gene in antisense orientation.

18. The recombinant influenza virus of claim 17, wherein the neraminidase gene in antisense orientation is attached to the hemaglutinin vRNA segment, and optionally another gene or reporter gene is encoded in a second tandem vRNA, preferably in conjunction with NS2.

19. A method for the production of recombinant influenza viruses as defined in claims 1 to 18 comprising
(a) RNA polymerase I synthesis of recombinant vRNAs *in vivo,* in antisense or in sense tandem design,
(b) followed by infection with an influenza carrier strain constructed to include flanking ribozyme target sequences in the corresponding viral RNA segment, and
(c) thereafter selective vRNA inactivation through ribozyme cleavage.

20. A pharmaceutical composition comprising a recombinant influenza virus according to claims 1 to 18, preferably a recombinant influenza virus of claims 16 to 18.

21. Use of a recombinant influenza virus according to claims 1 to 18, preferably a recombinant influenza virus of claims 16 to 18, for preparing a medicament for vaccination purposes.

22. The use according to claim 21, wherein the medicament
(a) is suitable against influenza and/or against other infections;
(b) is present in form of inactivated preparations; and/or
(c) is present in form of live recombinant viruses.

23. Use of a recombinant influenza virus according to claims 1 to 18 for preparing agents for somatic gene therapy.

24. Use of a recombinant influenza virus according to claims 1 to 18 for preparing agents, for transfer and expression of foreign genes into cells infected by such viruses.

25. Use of a recombinant influenza virus according to claims 1 to 18 for preparing agents for transfer and expression of RNA molecules into cells infected by such viruses.

26. The use of claim 24, wherein the RNA molecules to be expressed are antisense sequences or double-strand sequences relative to the target cell cellular mRNA molcules, and/or the agent is suitable for sequence-specific gene silencing, preferably by antisense RNA or RNA interference mechanisms.

27. The use according to claims 23 to 26, wherein the agents are applicable in *ex vivo* and *in vivo* application schemes.

28. A method for the production of proteins or glycoproteins which comprises utilizing a recombinant influenza virus according to claims 1 to 19 as expression vector.

29. The method of claim 28, wherein the production is performed in cell culture cells or in fertilized chicken eggs.

30. A method for preventing and/or treating influenza which comprises administering an effective amount of a recombinant influenza virus according to claims 1 to 18, preferably of a recombinant influenza virus according to claims 16 to 18, to the mammal to be treated.

31. A method for somatic gene therapy, which method comprises subjecting the organism to be treated with a recombinant influenza virus according to claims 1 to 18.

32. A method for transfer and expression of foreign genes into cells, and for transfer and expression of RNA molecules into cells, which method comprises infecting the cells with a recombinant influenza virus according to claims 1 to 18.

33. Use of a recombinant influenza virus according to claims 1 to 18 for preparing agents for immunotherapy, preferably for autologous immunotherapy.

34. A method for an immunotherapy which comprises *ex vivo* infection of immune cells, preferably dentritic cells, with a recombinant influenza virus according to claims 1 to 18, and introduction of the transduced cells into the patient.

35. A method for the induction of antibodies which comprises utilizing a recombinant influenza virus according to claims 1 to 18 as an immunogen.


SEQUENCE LISTING

<110> ARTEMIS PHARMACEUTICALS GmbH

<120> Recombinant Influenza Viruses with Bicistronic vRNAs Coding for
Two Genes in Tandem Arrangement

<130> 000746ep/JH/ml

<140>
<141>

<160> 24

<170> PatentIn Ver. 2.1

<210> 1
<211> 12
<212> RNA
<213> Influenza A virus

<400> 1
ccugcuuuug cu                                                    12

<210> 2
<211> 12
<212> RNA
<213> Influenza B virus

<400> 2
nnygcuucug cu                                                    12

<210> 3
<211> 12
<212> RNA
<213> Influenza C virus

<400> 3
ccugcuucug cu                                                    12

<210> 4
<211> 12
<212> RNA
<213> Artificial Sequence

<220>
<223> Description of Artificial Sequence: Modified
      influenza A 5'-sequence (pHL1104 and pHL1920)

<400> 4
ccuguuucua cu                                                          12

<210> 5
<211> 12
<212> RNA
<213> Artificial Sequence

<220>
<223> Description of Artificial Sequence: Modified
      influenza A 3'-sequence (pHL1948)

<400> 5
ccugguucuc cu                                                          12

<210> 6
<211> 13
<212> RNA
<213> Influenza A virus

<220>
<223> Description of Artificial Sequence: Modified
      influenza A 3'-sequence (pHL1948)

<400> 6
aguagaaaca agg                                                         13

<210> 7
<211> 13
<212> RNA
<213> Influenza B virus

<400> 7
aguagwaaca rnn                                                         13

<210> 8
<211> 13
<212> RNA
<213> Influenza C virus

<400> 8
agcaguagca agr                                                         13

<210> 9
<211> 13
<212> RNA
<213> Artificial Sequence

<220>
<223> Description of Artificial Sequence: Modified
      influenza A 5'-sequence (pHL1920)

<400> 9
agaagaauca agg                                                          13


<210> 10
<211> 21
<212> RNA
<213> Influenza A virus

<400> 10
aguagaaaca aggnnnuuuu u                                                 21


<210> 11
<211> 21
<212> RNA
<213> Artificial Sequence

<220>
<223> Description of Artificial Sequence: Modified
      influenza A 5'-sequence (pHL1920)

<400> 11
agaagaauca aggnnnuuuu u                                                 21


<210> 12
<211> 21
<212> RNA
<213> Influenza B virus

<400> 12
aguagwaaca rnnnnnuuuu u                                                 21


<210> 13
<211> 19
<212> RNA
<213> Artificial Sequence

<220>
<223> Description of Artificial Sequence: Modified
      influenza C 5'-sequence

<400> 13
aguaguaaca agrguuuuu                                                    19

<210> 14
<211> 15
<212> RNA
<213> Artificial Sequence

<220>
<223> Description of Artificial Sequence: Modified
      influenza A 3'-sequence (pHL1104 and pHL1920)

<400> 14
nnnccuguuu cuacu                                                    15


<210> 15
<211> 15
<212> RNA
<213> Artificial Sequence

<220>
<223> Description of Artificial Sequence: Modified
      influenza A 3'-sequence (pHL1948)

<400> 15
nnnccugguu cuccu                                                    15


<210> 16
<211> 15
<212> RNA
<213> Artificial Sequence

<220>
<223> Description of Artificial Sequence: Modified
      influenza B 3' sequence

<400> 16
nnnnnyguuu cuacu                                                    15


<210> 17
<211> 14
<212> RNA
<213> Artificial Sequence

<220>
<223> Description of Artificial Sequence: Modified
      influenza C 3'-sequence

<400> 17
ccccuguuuc uacu                                                     14

<210> 18
<211> 10
<212> DNA
<213> Influenza A virus

<400> 18
aggtacgttc                                                              10


<210> 19
<211> 32
<212> DNA
<213> Influenza A virus

<400> 19
gctgaaaaat gatcttcttg aaaattgcag gc                                     32


<210> 20
<211> 3888
<212> DNA
<213> Artificial Sequence

<220>
<223> Description of Artificial Sequence: pHL1920

<400> 20
cccaaaaaaa aaaaaaaaaa aaaaaaaaag agtccagagt ggccccgccg ttccgcgccg 60
gggggggggg ggggggggga cactttcgga catctggtcg acctccagca tcgggggaaa 120
aaaaaaaaac aaagtttcgc ccggagtact ggtcgacctc cgaagttggg ggggagtaga 180
aacagggtag ataatcactc actgagtgac atccacatcg cgagcgcgcg taatacgact 240
cactataggg cgaattgggt accgggcccc ccctcgaggt cgacggtatc gataagcttc 300
gacgagattt tcaggagcta aggaagctaa aatggagaaa aaaatcactg gatataccac 360
cgttgatata tcccaatggc atcgtaaaga acattttgag gcatttcagt cagttgctca 420
atgtacctat aaccagaccg ttcagctgga tattacggcc tttttaaaga ccgtaaagaa 480
aaataagcac aagttttatc cggcctttat tcacattctt gcccgcctga tgaatgctca 540
tccggaattc cgtatggcaa tgaaagacgg tgagctggtg atatgggata gtgttcaccc 600
ttgttacacc gttttccatg agcaaactga aacgttttca tcgctctgga gtgaatacca 660
cgacgatttc cggcagtttc tacacatata ttcgcaagat gtggcgtgtt acggtgaaaa 720
cctggcctat ttccctaaag ggtttattga gaatatgttt ttcgtctcag ccaatccctg 780
ggtgagtttc accagttttg atttaaacgt ggccaatatg gacaacttct tcgcccccgt 840
tttcaccatg ggcaaatatt atacgcaagg cgacaaggtg ctgatgccgc tggcgattca 900
ggttcatcat gccgtttgtg atggcttcca tgtcggcaga atgcttaatg aattacaaca 960
gtactgcgat gagtggcagg gcggggcgta atttttttaa ggcagttatt ggtgccctta 1020
aacgcctggt gctacgcctg aataagtgat aataagcgga tgaatggcag aaattcgtcg 1080
aagcttgata tcgaattcct gcagcccggg ggatccacta gttctagagc ggccgccacc 1140
gcggtggagc tccagctttt gttcccttta gtgagggtta attgcgcgca ggcctagcta 1200
ggtaaagaaa aatacccttg attcttctaa taacccggcg gcccaaaatg ccgactcgga 1260
gcgaaagata tacctccccc ggggccggga ggtcgcgtca ccgaccacgc cgccggccca 1320
ggcgacgcgc gacacggaca cctgtcccca aaaacgccac catcgcagcc acacacggag 1380
cgcccggggc cctctggtca accccaggac acacgcggga gcagcgccgg gccggggacg 1440
ccctcccggc cgcccgtgcc acacgcaggg ggccggcccg tgtctccaga gcgggagccg 1500
gaagcatttt cggccggccc ctcctacgac cgggacacac gagggaccga aggccggcca 1560

ggcgcgacct ctcgggccgc acgcgcgctc agggagcgct ctccgactcc gcacggggac 1620
tcgccagaaa ggatcgtgac ctgcattaat gaatcagggg ataacgcagg aaagaacatg 1680
tgagcaaaag gccagcaaaa ggccaggaac cgtaaaaagg ccgcgttgct ggcgtttttc 1740
cataggctcc gcccccctga cgagcatcac aaaaatcgac gctcaagtca gaggtggcga 1800
aacccgacag gactataaag ataccaggcg tttccccctg gaagctccct cgtgcgctct 1860
cctgttccga ccctgccgct taccggatac ctgtccgcct ttctcccttc gggaagcgtg 1920
gcgctttctc atagctcacg ctgtaggtat ctcagttcgg tgtaggtcgt tcgctccaag 1980
ctgggctgtg tgcacgaacc ccccgttcag cccgaccgct gcgccttatc cggtaactat 2040
cgtcttgagt ccaacccggt aagacacgac ttatcgccac tggcagcagc cactggtaac 2100
aggattagca gagcgaggta tgtaggcggt gctacagagt tcttgaagtg gtggcctaac 2160
tacggctaca ctagaaggac agtatttggt atctgcgctc tgctgaagcc agttaccttc 2220
ggaaaaagag ttggtagctc ttgatccggc aaacaaacca ccgctggtag cggtggtttt 2280
tttgtttgca agcagcagat tacgcgcaga aaaaaaggat ctcaagaaga tcctttgatc 2340
ttttctacgg ggtctgacgc tcagtggaac gaaaactcac gttaagggat tttggtcatg 2400
agattatcaa aaaggatctt cacctagatc cttttaaatt aaaaatgaag ttttaaatca 2460
atctaaagta tatatgagta aacttggtct gacagttacc aatgcttaat cagtgaggca 2520
cctatctcag cgatctgtct atttcgttca tccatagttg cctgactccc cgtcgtgtag 2580
ataactacga tacgggaggg cttaccatct ggccccagtg ctgcaatgat accgcgagac 2640
ccacgctcac cggctccaga tttatcagca ataaaccagc cagccggaag ggccgagcgc 2700
agaagtggtc ctgcaacttt atccgcctcc atccagtcta ttaattgttg ccgggaagct 2760
agagtaagta gttcgccagt taatagtttg cgcaacgttg ttgccattgc tacaggcatc 2820
gtggtgtcac gctcgtcgtt tggtatggct tcattcagct ccggttccca acgatcaagg 2880
cgagttacat gatcccccat gttgtgcaaa aaagcggtta gctccttcgg tcctccgatc 2940
gttgtcagaa gtaagttggc cgcagtgtta tcactcatgg ttatggcagc actgcataat 3000
tctcttactg tcatgccatc cgtaagatgc ttttctgtga ctggtgagta ctcaaccaag 3060
tcattctgag aatagtgtat gcggcgaccg agttgctctt gcccggcgtc aacacgggat 3120
aataccgcgc cacatagcag aactttaaaa gtgctcatca ttggaaaacg ttcttcgggg 3180
cgaaaactct caaggatctt accgctgttg agatccagtt cgatgtaacc cactcgtgca 3240
cccaactgat cttcagcatc ttttactttc accagcgttt ctgggtgagc aaaaacagga 3300
aggcaaaatg ccgcaaaaaa gggaataagg gcgacacgga aatgttgaat actcatactc 3360
ttcctttttc aatattattg aagcatttat cagggttatt gtctcatgag cggatacata 3420
tttgaatgta tttagaaaaa taaacaaaag agtttgtaga aacgcaaaaa ggccatccgt 3480
caggatggcc ttctgcttaa tttgatgcct ggcagtttat ggcgggcgtc ctgcccgcca 3540
ccctccgggc cgttgcttcg caacgttcaa atccgctccc ggcggatttg tcctactcag 3600
gagagcgttc accgacaaac aacagataaa acgaaaggcc cagtctttcg actgagcctt 3660
tcgttttatt tgatgcctgg cagttcccta ctctcgcatg gggagacccc acactaccat 3720
cggcgctacg gcgtttcact tctgagttcg gcatggggtc aggtgggacc accgcgctac 3780
tgccgccagg caaattctgt tttatcagac cgcttctgcg ttctgattta atctgtatca 3840
ggctgaaaat cttctctcat ccgccaaaac agaagctagc ggccgatc 3888

<210> 21
<211> 4500
<212> DNA
<213> Artificial Sequence

<220>
<223> Description of Artificial Sequence: pHL3196

<400> 21
agtagaaaca gggtagataa tcactcactg agtgacatcc acatcgcgag cgcgaaggta 60
cgttctcgag cgcgcgtaat acgactcact atagggcgaa ttgggtacgt tccatcatgg 120
agaaaaaaat cactggatat accaccgttg atatatccca atggcatcgt aaagaacatt 180

ttgaggcatt tcagtcagtt gctcaatgta cctataacca gaccgttcag ctggatatta 240
cggccttttt aaagaccgta aagaaaaata agcacaagtt ttatccggcc tttattcaca 300
ttcttgcccg cctgatgaat gctcatccgg aattccgtat ggcaatgaaa gacggtgagc 360
tggtgatatg ggatagtgtt cacccttgtt acaccgtttt ccatgagcaa actgaaacgt 420
tttcatcgct ctggagtgaa taccacgacg atttccggca gtttctacac atatattcgc 480
aagatgtggc gtgttacggt gaaaacctgg cctatttccc taaagggttt attgagaata 540
tgtttttcgt ctcagccaat ccctgggtga gtttcaccag ttttgattta aacgtggcca 600
atatggacaa cttcttcgcc cccgttttca ccatgggcaa atattatacg caaggcgaca 660
aggtgctgat gccgctggcg attcaggttc atcatgccgt ctgtgatggc ttccatgtcg 720
gcagaatgct taatgaatta caacagtact gcgatgagtg gcagggcggg gcgcgttaac 780
gagatcagct gaaaaatgat cttcttgaaa atttgcaggc cgtacgtgta ccgggccccc 840
cctcgactcg cgaaggagtc caccatgagt aaaggagaag aacttttcac tggagttgtc 900
ccaattcttg ttgaattaga tggtgatgtt aatgggcaca aattttctgt cagtggagag 960
ggtgaaggtg atgcaacata cggaaaactt acccttaaat ttatttgcac tactggaaaa 1020
ctacctgttc catggccaac acttgtcact actttcactt atggtgttca atgcttttca 1080
agatacccag atcatatgaa acagcatgac tttttcaaga gtgccatgcc cgaaggttat 1140
gtacaggaaa gaactatatt tttcaaagat gacgggaact acaagacacg tgctgaagtc 1200
aagtttgaag gtgataccct tgttaataga atcgagttaa aaggtattga ttttaaagaa 1260
gatggaaaca ttcttggaca caaattggaa tacaactata actcacacaa tgtatacatc 1320
atggctgaca agcagaagaa cggaatcaag gccaacttca agacccgcca caacatcgag 1380
gacggcggcg tgcagctggc cgaccactac cagcagaaca ccccaattgg cgatggccct 1440
gtccttttac cagacaacca ttacctgtcc acacaatctg ccctttcgaa agatcccaac 1500
gaaaagagag accacatggt ccttcttgag tttgtaacag ctgctgggat tacacatggc 1560
atggatgaac tatacaaggg atcccatcac catcaccatc actaagctcc atggtctaga 1620
tatcgatagg cctagctagg taaagaaaaa tacccttgtt tctactaata acccggcggc 1680
ccaaaatgcc gactcggagc gaaagatata cctcccccgg ggccgggagg tcgcgtcacc 1740
gaccacgccg ccggcccagg cgacgcgcga cacggacacc tgtccccaaa aacgccacca 1800
tcgcagccac acacggagcg cccggggccc tctggtcaac cccaggacac acgcgggagc 1860
agcgccgggc cggggacgcc ctcccggccg cccgtgccac acgcaggggg ccggcccgtg 1920
tctccagagc gggagccgga agcattttcg gccggcccct cctacgaccg ggacacacga 1980
gggaccgaag gccggccagg cgcgacctct cgggccgcac gcgcgctcag ggagcgctct 2040
ccgactccgc acggggactc gccagaaagg atcgtgacct gcattaatga atcaggggat 2100
aacgcaggaa agaacatgtg agcaaaaggc cagcaaaagg ccaggaaccg taaaaaggcc 2160
gcgttgctgg cgtttttcca taggctccgc ccccctgacg agcatcacaa aaatcgacgc 2220
tcaagtcaga ggtggcgaaa cccgacagga ctataaagat accaggcgtt tccccctgga 2280
agctccctcg tgcgctctcc tgttccgacc ctgccgctta ccggatacct gtccgccttt 2340
ctcccttcgg gaagcgtggc gctttctcat agctcacgct gtaggtatct cagttcggtg 2400
taggtcgttc gctccaagct gggctgtgtg cacgaacccc ccgttcagcc cgaccgctgc 2460
gccttatccg gtaactatcg tcttgagtcc aacccggtaa gacacgactt atcgccactg 2520
gcagcagcca ctggtaacag gattagcaga gcgaggtatg taggcggtgc tacagagttc 2580
ttgaagtggt ggcctaacta cggctacact agaaggacag tatttggtat ctgcgctctg 2640
ctgaagccag ttaccttcgg aaaaagagtt ggtagctctt gatccggcaa acaaaccacc 2700
gctggtagcg gtggtttttt tgtttgcaag cagcagatta cgcgcagaaa aaaaggatct 2760
caagaagatc ctttgatctt ttctacgggg tctgacgctc agtggaacga aaactcacgt 2820
taagggattt tggtcatgag attatcaaaa aggatcttca cctagatcct tttaaattaa 2880
aaatgaagtt ttaaatcaat ctaaagtata tatgagtaaa cttggtctga cagttaccaa 2940
tgcttaatca gtgaggcacc tatctcagcg atctgtctat ttcgttcatc catagttgcc 3000
tgactccccg tcgtgtagat aactacgata cgggagggct taccatctgg ccccagtgct 3060
gcaatgatac cgcgagaccc acgctcaccg gctccagatt tatcagcaat aaaccagcca 3120
gccggaaggg ccgagcgcag aagtggtcct gcaactttat ccgcctccat ccagtctatt 3180
aattgttgcc gggaagctag agtaagtagt tcgccagtta atagtttgcg caacgttgtt 3240
gccattgcta caggcatcgt ggtgtcacgc tcgtcgtttg gtatggcttc attcagctcc 3300
ggttcccaac gatcaaggcg agttacatga tcccccatgt tgtgcaaaaa agcggttagc 3360

tccttcggtc ctccgatcgt tgtcagaagt aagttggccg cagtgttatc actcatggtt 3420
atggcagcac tgcataattc tcttactgtc atgccatccg taagatgctt ttctgtgact 3480
ggtgagtact caaccaagtc attctgagaa tagtgtatgc ggcgaccgag ttgctcttgc 3540
ccggcgtcaa cacgggataa taccgcgcca catagcagaa ctttaaaagt gctcatcatt 3600
ggaaaacgtt cttcggggcg aaaactctca aggatcttac cgctgttgag atccagttcg 3660
atgtaaccca ctcgtgcacc caactgatct tcagcatctt ttactttcac cagcgtttct 3720
gggtgagcaa aaacaggaag gcaaaatgcc gcaaaaaagg gaataagggc gacacggaaa 3780
tgttgaatac tcatactctt cctttttcaa tattattgaa gcatttatca gggttattgt 3840
ctcatgagcg gatacatatt tgaatgtatt tagaaaaata aacaaaagag tttgtagaaa 3900
cgcaaaaagg ccatccgtca ggatggcctt ctgcttaatt tgatgcctgg cagtttatgg 3960
cgggcgtcct gcccgccacc ctccgggccg ttgcttcgca acgttcaaat ccgctcccgg 4020
cggatttgtc ctactcagga gagcgttcac cgacaaacaa cagataaaac gaaaggccca 4080
gtctttcgac tgagcctttc gttttatttg atgcctggca gttccctact ctcgcatggg 4140
gagaccccac actaccatcg gcgctacggc gtttcacttc tgagttcggc atggggtcag 4200
gtgggaccac cgcgctactg ccgccaggca aattctgttt tatcagaccg cttctgcgtt 4260
ctgatttaat ctgtatcagg ctgaaaatct tctctcatcc gccaaaacag aagctagcgg 4320
ccgatcccca aaaaaaaaaa aaaaaaaaaa aaaaagagtc cagagtggcc ccgccgttcc 4380
gcgccggggg gggggggggg gggggacact ttcggacatc tggtcgacct ccagcatcgg 4440
gggaaaaaaa aaaaacaaag tttcgcccgg agtactggtc gacctccgaa gttggggggg 4500

<210> 22
<211> 4721
<212> DNA
<213> Artificial Sequence

<220>
<223> Description of Artificial Sequence: pHL3224

<400> 22
atctagacca tggagcttag tgatggtgat ggtgatggga tcccttgtat agttcatcca 60
tgccatgtgt aatcccagca gctgttacaa actcaagaag gaccatgtgg tctctctttt 120
cgttgggatc tttcgaaagg gcagattgtg tggacaggta atggttgtct ggtaaaagga 180
cagggccatc gccaattggg gtgttctgct ggtagtggtc ggccagctgc acgccgccgt 240
cctcgatgtt gtggcgggtc ttgaagttgg ccttgattcc gttcttctgc ttgtcagcca 300
tgatgtatac attgtgtgag ttatagttgt attccaattt gtgtccaaga atgtttccat 360
cttctttaaa atcaatacct tttaactcga ttctattaac aagggtatca ccttcaaact 420
tgacttcagc acgtgtcttg tagttcccgt catctttgaa aaatatagtt ctttcctgta 480
cataaccttc gggcatggca ctcttgaaaa agtcatgctg tttcatatga tctgggtatc 540
ttgaaaagca ttgaacacca taagtgaaag tagtgacaag tgttggccat ggaacaggta 600
gttttccagt agtgcaaata aatttaaggg taagttttcc gtatgttgca tcaccttcac 660
cctctccact gacagaaaat ttgtgcccat taacatcacc atctaattca acaagaattg 720
ggacaactcc agtgaaaagt tcttctcctt tactcatggt ggactccttc gcgagtcgag 780
ggggggcccg gtacacgtac gcgctcgaga acgtaccttc gcgctcgcga tgtggatgtc 840
actcagtgag tgattatcta ccctgtttct actccccccc aacttcggag gtcgaccagt 900
actccgggcg aaactttgtt tttttttttt cccccgatgc tggaggtcga ccagatgtcc 960
gaaagtgtcc cccccccccc ccccccccgg cgcggaacgg cggggccact ctggactctt 1020
tttttttttt tttttttttt ttttggggat cggccgctag cttctgtttt ggcggatgag 1080
agaagatttt cagcctgata cagattaaat cagaacgcag aagcggtctg ataaaacaga 1140
atttgcctgg cggcagtagc gcggtggtcc cacctgaccc catgccgaac tcagaagtga 1200
aacgccgtag cgccgatggt agtgtggggt ctccccatgc gagagtaggg aactgccagg 1260
catcaaataa aacgaaaggc tcagtcgaaa gactgggcct ttcgttttat ctgttgtttg 1320
tcggtgaacg ctctcctgag taggacaaat ccgccgggag cggatttgaa cgttgcgaag 1380

caacggcccg gagggtggcg ggcaggacgc ccgccataaa ctgccaggca tcaaattaag 1440
cagaaggcca tcctgacgga tggccttttt gcgtttctac aaactctttt gtttattttt 1500
ctaaatacat tcaaatatgt atccgctcat gagacaataa ccctgataaa tgcttcaata 1560
atattgaaaa aggaagagta tgagtattca acatttccgt gtcgccctta ttcccttttt 1620
tgcggcattt tgccttcctg tttttgctca cccagaaacg ctggtgaaag taaaagatgc 1680
tgaagatcag ttgggtgcac gagtgggtta catcgaactg gatctcaaca gcggtaagat 1740
ccttgagagt tttcgccccg aagaacgttt tccaatgatg agcactttta aagttctgct 1800
atgtggcgcg gtattatccc gtgttgacgc cgggcaagag caactcggtc gccgcataca 1860
ctattctcag aatgacttgg ttgagtactc accagtcaca gaaaagcatc ttacggatgg 1920
catgacagta agagaattat gcagtgctgc cataaccatg agtgataaca ctgcggccaa 1980
cttacttctg acaacgatcg gaggaccgaa ggagctaacc gcttttttgc acaacatggg 2040
ggatcatgta actcgccttg atcgttggga accggagctg aatgaagcca taccaaacga 2100
cgagcgtgac accacgatgc ctgtagcaat ggcaacaacg ttgcgcaaac tattaactgg 2160
cgaactactt actctagctt cccggcaaca attaatagac tggatggagg cggataaagt 2220
tgcaggacca cttctgcgct cggcccttcc ggctggctgg tttattgctg ataaatctgg 2280
agccggtgag cgtgggtctc gcggtatcat tgcagcactg gggccagatg gtaagccctc 2340
ccgtatcgta gttatctaca cgacggggag tcaggcaact atggatgaac gaaatagaca 2400
gatcgctgag ataggtgcct cactgattaa gcattggtaa ctgtcagacc aagtttactc 2460
atatatactt tagattgatt taaaacttca tttttaattt aaaaggatct aggtgaagat 2520
cctttttgat aatctcatga ccaaaatccc ttaacgtgag ttttcgttcc actgagcgtc 2580
agaccccgta gaaaagatca aaggatcttc ttgagatcct ttttttctgc gcgtaatctg 2640
ctgcttgcaa acaaaaaaac caccgctacc agcggtggtt tgtttgccgg atcaagagct 2700
accaactctt tttccgaagg taactggctt cagcagagcg cagataccaa atactgtcct 2760
tctagtgtag ccgtagttag gccaccactt caagaactct gtagcaccgc ctacatacct 2820
cgctctgcta atcctgttac cagtggctgc tgccagtggc gataagtcgt gtcttaccgg 2880
gttggactca agacgatagt taccggataa ggcgcagcgg tcgggctgaa cggggggttc 2940
gtgcacacag cccagcttgg agcgaacgac ctacaccgaa ctgagatacc tacagcgtga 3000
gctatgagaa agcgccacgc ttcccgaagg gagaaaggcg gacaggtatc cggtaagcgg 3060
cagggtcgga acaggagagc gcacgaggga gcttccaggg ggaaacgcct ggtatcttta 3120
tagtcctgtc gggtttcgcc acctctgact tgagcgtcga tttttgtgat gctcgtcagg 3180
ggggcggagc ctatggaaaa acgccagcaa cgcggccttt ttacggttcc tggccttttg 3240
ctggcctttt gctcacatgt tctttcctgc gttatcccct gattcattaa tgcaggtcac 3300
gatcctttct ggcgagtccc cgtgcggagt cggagagcgc tccctgagcg cgcgtgcggc 3360
ccgagaggtc gcgcctggcc ggccttcggt ccctcgtgtg tcccggtcgt aggaggggcc 3420
ggccgaaaat gcttccggct cccgctctgg agacacgggc cggccccctg cgtgtggcac 3480
gggcggccgg gagggcgtcc ccggcccggc gctgctcccg cgtgtgtcct ggggttgacc 3540
agagggcccc gggcgctccg tgtgtggctg cgatggtggc gtttttgggg acaggtgtcc 3600
gtgtcgcgcg tcgcctgggc cggcggcgtg gtcggtgacg cgacctcccg gccccggggg 3660
aggtatatct ttcgctccga gtcggcattt tgggccgccg ggttattagt agaaacaagg 3720
gtatttttct ttacctagct aggcctgcgc gcaattaacc ctcactaaag ggaacaaaag 3780
ctggagctcc accgcggtgg cggccgctct agaactagtg gatcccccgg gctgcaggaa 3840
ttcgatatca agcttcgacg aatttctgcc attcatccgc ttattatcac ttattcaggc 3900
gtagcaccag gcgtttaagg gcaccaataa ctgccttaaa aaaattacgc cccgccctgc 3960
cactcatcgc agtactgttg taattcatta agcattctgc cgacatggaa gccatcacaa 4020
acggcatgat gaacctgaat cgccagcggc atcagcacct tgtcgccttg cgtataatat 4080
ttgcccatgg tgaaaacggg ggcgaagaag ttgtccatat tggccacgtt taaatcaaaa 4140
ctggtgaaac tcacccaggg attggctgag acgaaaaaca tattctcaat aaacccttta 4200
gggaaatagg ccaggttttc accgtaacac gccacatctt gcgaatatat gtgtagaaac 4260
tgccggaaat cgtcgtggta ttcactccag agcgatgaaa acgtttcagt ttgctcatgg 4320
aaaacggtgt aacaagggtg aacactatcc catatcacca gctcaccgtc tttcattgcc 4380
atacggaatt ccggatgagc attcatcagg cgggcaagaa tgtgaataaa ggccggataa 4440
aacttgtgct tatttttctt tacggtcttt aaaaaggccg taatatccag ctgaacggtc 4500
tggttatagg tacattgagc aactgactga aatgcctcaa aatgttcttt acgatgccat 4560

tgggatatat caacggtggt atatccagtg atttttttct ccattttagc ttccttagct 4620
cctgaaaatc tcgtcgaagc ttatcgatac cgtcgacctc gagggggggc ccggtacggc 4680
ctgcaaattt tcaagaagat catttttcag ctgatctcgt t 4721

<210> 23
<211> 5517
<212> DNA
<213> Artificial Sequence

<220>
<223> Description of Artificial Sequence: pHL3235

<400> 23
agtagaaaca gggtagataa tcactcactg agtgacatcc acatcgcgag cgcgaaggta 60
cgttctcgag cgcgcgtaat acgactcact atagggcgaa ttgggtacgt tccatcatgg 120
agaaaaaaat cactggatat accaccgttg atatatccca atggcatcgt aaagaacatt 180
ttgaggcatt tcagtcagtt gctcaatgta cctataacca gaccgttcag ctggatatta 240
cggccttttt aaagaccgta aagaaaaata agcacaagtt ttatccggcc tttattcaca 300
ttcttgcccg cctgatgaat gctcatccgg aattccgtat ggcaatgaaa gacggtgagc 360
tggtgatatg ggatagtgtt cacccttgtt acaccgtttt ccatgagcaa actgaaacgt 420
tttcatcgct ctggagtgaa taccacgacg atttccggca gtttctacac atatattcgc 480
aagatgtggc gtgttacggt gaaaacctgg cctatttccc taaagggttt attgagaata 540
tgtttttcgt ctcagccaat ccctgggtga gtttcaccag ttttgattta aacgtggcca 600
atatggacaa cttcttcgcc cccgttttca ccatgggcaa atattatacg caaggcgaca 660
aggtgctgat gccgctggcg attcaggttc atcatgccgt ctgtgatggc ttccatgtcg 720
gcagaatgct taatgaatta caacagtact gcgatgagtg gcagggcggg gcgcgttaac 780
gagatcagct gaaaaatgat cttcttgaaa atttgcaggc cgtacgtgta ccgggccccc 840
cctcgactcg cgaaggagtc caccatgagt aaaggagaag aacttttcac tggagttgtc 900
ccaattcttg ttgaattaga tggtgatgtt aatgggcaca aattttctgt cagtggagag 960
ggtgaaggtg atgcaacata cggaaaactt acccttaaat ttatttgcac tactggaaaa 1020
ctacctgttc catggccaac acttgtcact actttcactt atggtgttca atgcttttca 1080
agatacccag atcatatgaa acagcatgac tttttcaaga gtgccatgcc cgaaggttat 1140
gtacaggaaa gaactatatt tttcaaagat gacgggaact acaagacacg tgctgaagtc 1200
aagtttgaag gtgataccct tgttaataga atcgagttaa aaggtattga ttttaaagaa 1260
gatggaaaca ttcttggaca caaattggaa tacaactata actcacacaa tgtatacatc 1320
atggctgaca agcagaagaa cggaatcaag gccaacttca agacccgcca caacatcgag 1380
gacggcggcg tgcagctggc cgaccactac cagcagaaca ccccaattgg cgatggccct 1440
gtccttttac cagacaacca ttacctgtcc acacaatctg ccctttcgaa agatcccaac 1500
gaaaagagag accacatggt ccttcttgag tttgtaacag ctgctgggat tacacatggc 1560
atggatgaac tatacaaggg atcttcatga tctcagcaaa ctcttccttc ttaatccttc 1620
cagactcgaa gtcaattcgt gcatcaatcc gggccctaga caccatggcc tccaccatac 1680
tggaaattcc aactggtctt ctgtatgagc tgctagggaa gaatttctcg aataggttgc 1740
aacacttctg gtacatttgt tcatcctcaa ggattcccct ttgactcgta ttgagaatgg 1800
aacggtttct cttagggatc caagagtgtg tagttgccac agcatcatat tccatgcttt 1860
tggctggacc atgggctggc attaccgcag cattgtttac agattcaatt tccttatgac 1920
tgacaaacgg gttcatggga ttacaaagtc ttccctgata gtcttcatcc attagttccc 1980
atttcaggca aacttccggg atgtggagat tccgaatgtt gtacaggttt ggtccgccat 2040
ctgaaaccaa cagtcctgcc tttgagcggg tctgctccca cagcttcttt agctcgaatg 2100
acctcctcgt ttggatttgt gtgtctcccc tgtgacaccg gtatgtatat ctgtagtcct 2160
tgatgaataa ttggagagcc atttgggctg ttgccggtcc aagatcattg tttatcatgt 2220
tattctttat cactgttact ccaatgctca tatcagccga ttcattaatt cctgatactc 2280
caaagctggg caactccata ctaaaattgg ctacaaatcc atagcggtag aaaaagcttg 2340

tgaattcgaa tgttcctgtc ctatttatat aggacttttt cttgctcata ttgatcccaa 2400
ctagcttgca ggttctgtag aatctatcca ctcccgcttg tattccctca tgatttggtg 2460
cattcacgat gagagcaaaa tcatcagagg actgaagtcc atcccaccag tatgtggttt 2520
tggtgtatct cttttgccca agattcagga ttgagactcc caacactgta ctcagcatgt 2580
tgaacatacc catcatcatt cccgggctta atgaggctgt gccgtctatt atgagaggat 2640
cgataggcct agctaggtaa agaaaaatac ccttgtttct actaataacc cggcggccca 2700
aaatgccgac tcggagcgaa agatatacct cccccggggc cgggaggtcg cgtcaccgac 2760
cacgccgccg gcccaggcga cgcgcgacac ggacacctgt ccccaaaaac gccaccatcg 2820
cagccacaca cggagcgccc ggggccctct ggtcaacccc aggacacacg cgggagcagc 2880
gccgggccgg ggacgccctc ccggccgccc gtgccacacg cagggggccg gcccgtgtct 2940
ccagagcggg agccggaagc attttcggcc ggcccctcct acgaccggga cacacgaggg 3000
accgaaggcc ggccaggcgc gacctctcgg gccgcacgcg cgctcaggga gcgctctccg 3060
actccgcacg gggactcgcc agaaaggatc gtgacctgca ttaatgaatc aggggataac 3120
gcaggaaaga acatgtgagc aaaaggccag caaaaggcca ggaaccgtaa aaaggccgcg 3180
ttgctggcgt ttttccatag gctccgcccc cctgacgagc atcacaaaaa tcgacgctca 3240
agtcagaggt ggcgaaaccc gacaggacta taaagatacc aggcgtttcc ccctggaagc 3300
tccctcgtgc gctctcctgt tccgaccctg ccgcttaccg gatacctgtc cgcctttctc 3360
ccttcgggaa gcgtggcgct ttctcatagc tcacgctgta ggtatctcag ttcggtgtag 3420
gtcgttcgct ccaagctggg ctgtgtgcac gaaccccccg ttcagcccga ccgctgcgcc 3480
ttatccggta actatcgtct tgagtccaac ccggtaagac acgacttatc gccactggca 3540
gcagccactg gtaacaggat tagcagagcg aggtatgtag gcggtgctac agagttcttg 3600
aagtggtggc ctaactacgg ctacactaga aggacagtat ttggtatctg cgctctgctg 3660
aagccagtta ccttcggaaa aagagttggt agctcttgat ccggcaaaca aaccaccgct 3720
ggtagcggtg gtttttttgt ttgcaagcag cagattacgc gcagaaaaaa aggatctcaa 3780
gaagatcctt tgatcttttc tacggggtct gacgctcagt ggaacgaaaa ctcacgttaa 3840
gggattttgg tcatgagatt atcaaaaagg atcttcacct agatcctttt aaattaaaaa 3900
tgaagtttta aatcaatcta aagtatatat gagtaaactt ggtctgacag ttaccaatgc 3960
ttaatcagtg aggcacctat ctcagcgatc tgtctatttc gttcatccat agttgcctga 4020
ctccccgtcg tgtagataac tacgatacgg gagggcttac catctggccc cagtgctgca 4080
atgataccgc gagacccacg ctcaccggct ccagatttat cagcaataaa ccagccagcc 4140
ggaagggccg agcgcagaag tggtcctgca actttatccg cctccatcca gtctattaat 4200
tgttgccggg aagctagagt aagtagttcg ccagttaata gtttgcgcaa cgttgttgcc 4260
attgctacag gcatcgtggt gtcacgctcg tcgtttggta tggcttcatt cagctccggt 4320
tcccaacgat caaggcgagt tacatgatcc cccatgttgt gcaaaaaagc ggttagctcc 4380
ttcggtcctc cgatcgttgt cagaagtaag ttggccgcag tgttatcact catggttatg 4440
gcagcactgc ataattctct tactgtcatg ccatccgtaa gatgcttttc tgtgactggt 4500
gagtactcaa ccaagtcatt ctgagaatag tgtatgcggc gaccgagttg ctcttgcccg 4560
gcgtcaacac gggataatac cgcgccacat agcagaactt taaaagtgct catcattgga 4620
aaacgttctt cggggcgaaa actctcaagg atcttaccgc tgttgagatc cagttcgatg 4680
taacccactc gtgcacccaa ctgatcttca gcatctttta ctttcaccag cgtttctggg 4740
tgagcaaaaa caggaaggca aaatgccgca aaaaagggaa taagggcgac acggaaatgt 4800
tgaatactca tactcttcct ttttcaatat tattgaagca tttatcaggg ttattgtctc 4860
atgagcggat acatatttga atgtatttag aaaaataaac aaaagagttt gtagaaacgc 4920
aaaaaggcca tccgtcagga tggccttctg cttaatttga tgcctggcag tttatggcgg 4980
gcgtcctgcc cgccaccctc cgggccgttg cttcgcaacg ttcaaatccg ctcccggcgg 5040
atttgtccta ctcaggagag cgttcaccga caaacaacag ataaaacgaa aggcccagtc 5100
tttcgactga gcctttcgtt ttatttgatg cctggcagtt ccctactctc gcatggggag 5160
accccacact accatcggcg ctacggcgtt tcacttctga gttcggcatg gggtcaggtg 5220
ggaccaccgc gctactgccg ccaggcaaat tctgttttat cagaccgctt ctgcgttctg 5280
atttaatctg tatcaggctg aaaatcttct ctcatccgcc aaaacagaag ctagcggccg 5340
atccccaaaa aaaaaaaaaa aaaaaaaaaa aagagtccag agtggccccg ccgttccgcg 5400
ccgggggggg gggggggggg ggacactttc ggacatctgg tcgacctcca gcatcggggg 5460
aaaaaaaaaa aacaaagttt cgcccggagt actggtcgac ctccgaagtt gggggg 5517

<210> 24
<211> 5699
<212> DNA
<213> Artificial Sequence

<220>
<223> Description of Artificial Sequence: pHL3236

<400> 24
cctctcataa tagacggcac agcctcatta agcccgggaa tgatgatggg tatgttcaac 60
atgctgagta cagtgttggg agtctcaatc ctgaatcttg ggcaaaagag atacaccaaa 120
accacatact ggtgggatgg acttcagtcc tctgatgatt ttgctctcat cgtgaatgca 180
ccaaatcatg agggaataca agcgggagtg gatagattct acagaacctg caagctagtt 240
gggatcaata tgagcaagaa aaagtcctat ataaatagga caggaacatt cgaattcaca 300
agctttttct accgctatgg atttgtagcc aattttagta tggagttgcc cagctttgga 360
gtatcaggaa ttaatgaatc ggctgatatg agcattggag taacagtgat aaagaataac 420
atgataaaca atgatcttgg accggcaaca gcccaaatgg ctctccaatt attcatcaag 480
gactacagat atacataccg gtgtcacagg ggagacacac aaatccaaac gaggaggtca 540
ttcgagctaa agaagctgtg ggagcagacc cgctcaaagg caggactgtt ggtttcagat 600
ggcggaccaa acctgtacaa cattcggaat ctccacatcc cggaagtttg cctgaaatgg 660
gaactaatgg atgaagacta tcagggaaga ctttgtaatc ccatgaaccc gtttgtcagt 720
cataaggaaa ttgaatctgt aaacaatgct gcggtaatgc cagcccatgg tccagccaaa 780
agcatggaat atgatgctgt ggcaactaca cactcttgga tccctaagag aaaccgttcc 840
attctcaata cgagtcaaag gggaatcctt gaggatgaac aaatgtacca gaagtgttgc 900
aacctattcg agaaattctt ccctagcagc tcatacagaa gaccagttgg aatttccagt 960
atggtggagg ccatggtgtc tagggcccgg attgatgcac gaattgactt cgagtctgga 1020
aggattaaga aggaagagtt tgctgagatc atgaagatcc cccgggctgc aggaattcga 1080
tatcaagctt cgacgaattt ctgccattca tccgcttatt atcacttatt caggcgtagc 1140
accaggcgtt taagggcacc aataactgcc ttaaaaaaat tacgccccgc cctgccactc 1200
atcgcagtac tgttgtaatt cattaagcat tctgccgaca tggaagccat cacaaacggc 1260
atgatgaacc tgaatcgcca gcggcatcag caccttgtcg ccttgcgtat aatatttgcc 1320
catggtgaaa acgggggcga agaagttgtc catattggcc acgtttaaat caaaactggt 1380
gaaactcacc cagggattgg ctgagacgaa aaacatattc tcaataaacc ctttagggaa 1440
ataggccagg ttttcaccgt aacacgccac atcttgcgaa tatatgtgta gaaactgccg 1500
gaaatcgtcg tggtattcac tccagagcga tgaaaacgtt tcagtttgct catggaaaac 1560
ggtgtaacaa gggtgaacac tatcccatat caccagctca ccgtctttca ttgccatacg 1620
gaattccgga tgagcattca tcaggcgggc aagaatgtga ataaaggccg gataaaactt 1680
gtgcttattt ttctttacgg tctttaaaaa ggccgtaata tccagctgaa cggtctggtt 1740
ataggtacat tgagcaactg actgaaatgc ctcaaaatgt tctttacgat gccattggga 1800
tatatcaacg gtggtatatc cagtgatttt tttctccatt ttagcttcct tagctcctga 1860
aaatctcgtc gaagcttatc gataccgtcg acctcgaggg ggggcccggt acggcctgca 1920
aattttcaag aagatcattt ttcagctgat ctcgttatct agaccatgga gcttagtgat 1980
ggtgatggtg atgggatccc ttgtatagtt catccatgcc atgtgtaatc ccagcagctg 2040
ttacaaactc aagaaggacc atgtggtctc tcttttcgtt gggatctttc gaaagggcag 2100
attgtgtgga caggtaatgg ttgtctggta aaaggacagg gccatcgcca attggggtgt 2160
tctgctggta gtggtcggcc agctgcacgc cgccgtcctc gatgttgtgg cgggtcttga 2220
agttggcctt gattccgttc ttctgcttgt cagccatgat gtatacattg tgtgagttat 2280
agttgtattc caatttgtgt ccaagaatgt ttccatcttc tttaaaatca atacctttta 2340
actcgattct attaacaagg gtatcacctt caaacttgac ttcagcacgt gtcttgtagt 2400
tcccgtcatc tttgaaaaat atagttcttt cctgtacata accttcgggc atggcactct 2460
tgaaaaagtc atgctgtttc atatgatctg ggtatcttga aaagcattga acaccataag 2520

tgaaagtagt gacaagtgtt ggccatggaa caggtagttt tccagtagtg caaataaatt 2580
taagggtaag ttttccgtat gttgcatcac cttcaccctc tccactgaca gaaaatttgt 2640
gcccattaac atcaccatct aattcaacaa gaattgggac aactccagtg aaaagttctt 2700
ctcctttact catggtggac tccttcgcga gtcgaggggg ggcccggtac acgtacgcgc 2760
tcgagaacgt accttcgcgc tcgcgatgtg gatgtcactc agtgagtgat tatctaccct 2820
gtttctactc ccccccaact tcggaggtcg accagtactc cgggcgaaac tttgtttttt 2880
tttttttccc cgatgctgga ggtcgaccag atgtccgaaa gtgtcccccc cccccccccc 2940
ccccggcgcg gaacggcggg gccactctgg actctttttt tttttttttt tttttttttt 3000
ggggatcggc cgctagcttc tgttttggcg gatgagagaa gattttcagc ctgatacaga 3060
ttaaatcaga acgcagaagc ggtctgataa aacagaattt gcctggcggc agtagcgcgg 3120
tggtcccacc tgaccccatg ccgaactcag aagtgaaacg ccgtagcgcc gatggtagtg 3180
tggggtctcc ccatgcgaga gtagggaact gccaggcatc aaataaaacg aaaggctcag 3240
tcgaaagact gggcctttcg ttttatctgt tgtttgtcgg tgaacgctct cctgagtagg 3300
acaaatccgc cgggagcgga tttgaacgtt gcgaagcaac ggcccggagg gtggcgggca 3360
ggacgcccgc cataaactgc caggcatcaa attaagcaga aggccatcct gacggatggc 3420
ctttttgcgt ttctacaaac tcttttgttt atttttctaa atacattcaa atatgtatcc 3480
gctcatgaga caataaccct gataaatgct tcaataatat tgaaaaagga agagtatgag 3540
tattcaacat ttccgtgtcg cccttattcc cttttttgcg gcattttgcc ttcctgtttt 3600
tgctcaccca gaaacgctgg tgaaagtaaa agatgctgaa gatcagttgg gtgcacgagt 3660
gggttacatc gaactggatc tcaacagcgg taagatcctt gagagttttc gccccgaaga 3720
acgttttcca atgatgagca cttttaaagt tctgctatgt ggcgcggtat tatcccgtgt 3780
tgacgccggg caagagcaac tcggtcgccg catacactat tctcagaatg acttggttga 3840
gtactcacca gtcacagaaa agcatcttac ggatggcatg acagtaagag aattatgcag 3900
tgctgccata accatgagtg ataacactgc ggccaactta cttctgacaa cgatcggagg 3960
accgaaggag ctaaccgctt ttttgcacaa catgggggat catgtaactc gccttgatcg 4020
ttgggaaccg gagctgaatg aagccatacc aaacgacgag cgtgacacca cgatgcctgt 4080
agcaatggca acaacgttgc gcaaactatt aactggcgaa ctacttactc tagcttcccg 4140
gcaacaatta atagactgga tggaggcgga taaagttgca ggaccacttc tgcgctcggc 4200
ccttccggct ggctggttta ttgctgataa atctggagcc ggtgagcgtg ggtctcgcgg 4260
tatcattgca gcactggggc cagatggtaa gccctcccgt atcgtagtta tctacacgac 4320
ggggagtcag gcaactatgg atgaacgaaa tagacagatc gctgagatag gtgcctcact 4380
gattaagcat tggtaactgt cagaccaagt ttactcatat atactttaga ttgatttaaa 4440
acttcatttt taatttaaaa ggatctaggt gaagatcctt tttgataatc tcatgaccaa 4500
aatcccttaa cgtgagtttt cgttccactg agcgtcagac cccgtagaaa agatcaaagg 4560
atcttcttga gatccttttt ttctgcgcgt aatctgctgc ttgcaaacaa aaaaaccacc 4620
gctaccagcg gtggtttgtt tgccggatca agagctacca actctttttc cgaaggtaac 4680
tggcttcagc agagcgcaga taccaaatac tgtccttcta gtgtagccgt agttaggcca 4740
ccacttcaag aactctgtag caccgcctac atacctcgct ctgctaatcc tgttaccagt 4800
ggctgctgcc agtggcgata agtcgtgtct taccgggttg gactcaagac gatagttacc 4860
ggataaggcg cagcggtcgg gctgaacggg gggttcgtgc acacagccca gcttggagcg 4920
aacgacctac accgaactga gatacctaca gcgtgagcta tgagaaagcg ccacgcttcc 4980
cgaagggaga aaggcggaca ggtatccggt aagcggcagg gtcggaacag gagagcgcac 5040
gagggagctt ccagggggaa acgcctggta tctttatagt cctgtcgggt ttcgccacct 5100
ctgacttgag cgtcgatttt tgtgatgctc gtcagggggg cggagcctat ggaaaaacgc 5160
cagcaacgcg gcctttttac ggttcctggc cttttgctgg ccttttgctc acatgttctt 5220
tcctgcgtta tcccctgatt cattaatgca ggtcacgatc ctttctggcg agtccccgtg 5280
cggagtcgga gagcgctccc tgagcgcgcg tgcggcccga gaggtcgcgc ctggccggcc 5340
ttcggtccct cgtgtgtccc ggtcgtagga ggggccggcc gaaaatgctt ccggctcccg 5400
ctctggagac acgggccggc cccctgcgtg tggcacgggc ggccgggagg gcgtccccgg 5460
cccggcgctg ctcccgcgtg tgtcctgggg ttgaccagag ggccccgggc gctccgtgtg 5520
tggctgcgat ggtggcgttt ttggggacag gtgtccgtgt cgcgcgtcgc ctgggccggc 5580
ggcgtggtcg gtgacgcgac ctcccggccc cgggggaggt atatctttcg ctccgagtcg 5640
gcattttggg ccgccgggtt attagtagaa acaaggtatt ttttctttac ctagctagg 5699